# EUROPEAN PATENT SPECIFICATION

(11) **EP 3 141 555 B1**
(45) Date of publication and mention of the grant of the patent: **02.12.2020**
(21) Application number: 16801940.4
(22) Date of filing: 31.05.2016
(51) Int. Cl.: C07H 1/00, C07H 13/06

(54) **METHOD FOR PRODUCING SUCROSE FATTY ACID ESTER**
VERFAHREN ZUR HERSTELLUNG VON SACCHAROSEFETTSÄUREESTERN
PROCÉDÉ DE PRODUCTION D'ESTER D'ACIDE GRAS DE SACCHAROSE

(30) Priority: 01.06.2015 JP 2015111457; 17.02.2016 JP 2016027481; 30.05.2016 JP 2016107204
(43) Date of publication of application: 15.03.2017
(73) Proprietor: Microwave Chemical Co., Ltd., Osaka 5590025 (JP)
(72) Inventor: KIDANI, Keiji, Osaka-shi Osaka 5590025 (JP); AKIYAMA, Koki, Osaka-shi Osaka 5590025 (JP); KURIHARA, Hideshi, Osaka-shi Osaka 5590025 (JP); TSUKAHARA, Yasunori, Osaka-shi Osaka 5590025 (JP); OKUMURA, Haruki, Kusatsu-shi Shiga 5250061 (JP)
(74) Representative: Betten & Resch
(86) International application number: PCT/JP2016/065971
(87) International publication number: WO 2016/194887

(56) References cited:
- EP-A1- 0 254 376
- WO-A1-2006/046047
- WO-A1-2011/035853
- GB-A- 2 361 918
- JP-A- H1 017 588
- JP-A- S63 119 493
- JP-A- 2010 516 748
- JP-A- 2013 505 275
- JP-B1- 5 753 600
- REAL-FERNANDEZ,F. ET AL.: 'Microwave-assisted reaction of glycosylamine with aspartic acid' AMINO ACIDS vol. 39, no. 2, 2010, ISSN 0939-4451 pages 599 - 604, XP019853800
- GARCIA-MARTIN,F. ET AL.: 'Novel coupling method for the incorporation of 'complex' amino acid' PEPTIDE SCIENCE 2010, page 204, XP009500316
- GELO-PUJIC,M. ET AL.: 'Lipase-catalysed esterification of some alpha-D-glucopyranosides in dry media under focused microwave irradiation' J. CHEM. SOC. PERKIN TRANS.1 no. 23, 1996, ISSN 1472-7781 pages 2777 - 2780, XP055334265
- ZIAULLAH,H.P.V.R.: 'An efficient microwave- assisted enzyme-catalyzed regioselective synthesis of long chain acylated derivatives of flavonoid glycosides' TETRAHEDRON LETTERS vol. 54, no. 15, 2013, ISSN 0040-4039 pages 1933 - 1937, XP028998240

## Description

### Technical Field

The present invention relates to a method for producing a sucrose fatty acid ester having a high monoester selectivity.

### Background Art

Conventionally, a sugar fatty acid ester is produced by performing transesterification between a sugar and a fatty acid ester under standard heating (see Patent Document 1,

EP0254376 for example) or through microwave irradiation in the absence of any solvent (WO2006/046047 or GB2361918).

### Citation List

### Patent Document

Patent Document 1: JP S63-119493A

### Summary of Invention

### Technical Problem

In production of such a sugar fatty acid ester, a diester (di-form), a triester (tri-form), and the like are also produced together with a monoester (mono-form). Since a monoester of a sugar fatty acid ester such as a sucrose fatty acid ester (sugar ester) and a glucose fatty acid ester has excellent characteristics, there is a demand for production of a sugar fatty acid ester having a high monoester content. Note that a monoester of a sucrose fatty acid ester is known to have, for example, antimicrobial properties and thermal stability.

Furthermore, since such a monoester is used as an additive (e.g., emulsifier) to foods, cosmetics, drugs, and the like, there is also a demand for production of a sucrose fatty acid ester with no odoring.

The present invention was made in these circumstances, and it is an object thereof to provide a method for producing a sucrose fatty acid ester having a high monoester proportion and with no odoring.

### Solution to Problem

The present inventors conducted an in-depth study in order to achieve the above-described object, and found that it is possible to produce a sucrose fatty acid ester having a high monoester proportion and with no odoring, by performing heating through microwave irradiation such that the temperature is below the sucrose decomposition temperature, and thus the present invention was completed.

That is to say, the present invention is as follows.
[1] A method for producing a sucrose fatty acid ester, including: a step of preparing an aqueous solution containing sucrose and a basic catalyst; and a step of producing a sucrose fatty acid ester, by mixing the aqueous solution obtained in the aforementioned step, a fatty acid alkali metal salt, and a fatty acid ester, and heating the mixture with stirring under reduced pressure, wherein the step of producing a sucrose fatty acid ester has an earlier step of removing water from the mixture, and a subsequent step of performing transesterification after the earlier step, and in the subsequent step, heating is performed through microwave irradiation such that the temperature of the mixture is below a decomposition temperature of the sucrose.
[2] The method for producing a sucrose fatty acid ester according to [1], wherein a sucrose fatty acid ester with a monoester weight proportion of 60% by weight or more is produced.
[3] The method for producing a sucrose fatty acid ester according to [1] or [2], wherein the fatty acid ester is a fatty acid ester whose constituent fatty acid includes at least one selected from the group consisting of saturated and unsaturated fatty acids with 8 to 24 carbon atoms.
[4] The method for producing a sucrose fatty acid ester according to any one of [1] to [3], further including: a step of producing a fatty acid alkali metal salt by mixing and heating a fatty acid ester and a solution in which an alkali metal salt is dissolved in solvent, wherein the fatty acid alkali metal salt used in the step of producing a sucrose fatty acid ester has been produced in the step of producing a fatty acid alkali metal salt.
[5] The method for producing a sucrose fatty acid ester according to any one of [1] to [4], wherein, in the subsequent step, the heating is performed such that the temperature of the mixture is at or above a temperature 25°C lower than the decomposition temperature, and is below the decomposition temperature.
[6] The method for producing a sucrose fatty acid ester according to any one of [1] to [5], wherein, in the subsequent step, the heating is performed such that the temperature of the mixture increases.
[7] The method for producing a sucrose fatty acid ester according to any one of [1] to [6], wherein the heating time of the subsequent step is 6 hours or less.

### Advantageous Effects of Invention

With the method for producing a sucrose fatty acid ester according to the present invention, it is possible to produce a sucrose fatty acid ester having a high monoester proportion and with no odoring, by performing heating through microwave irradiation such that the temperature is below the sucrose decomposition temperature.

### Brief Description of Drawings

FIG. 1 is a table showing results of examples and comparative examples.
FIG. 2 is a graph showing time-series changes in monoester yields and monoester weight proportions of the examples and the comparative examples.
FIG. 3 is a table showing results of examples and comparative examples.
FIG. 4 is a graph showing time-series changes in monoester yields and monoester weight proportions of the examples and the comparative examples.
FIG. 5 is a table showing results of examples and comparative examples.
FIG. 6 is a graph showing time-series changes in monoester yields and monoester weight proportions of the examples and the comparative examples.
FIG. 7 is a table showing results of examples and comparative examples.
FIG. 8 is a graph showing time-series changes in monoester yields and monoester weight proportions of the examples and the comparative examples.
FIG. 9 is a table showing results of examples and comparative examples.
FIG. 10 is a graph showing time-series changes in monoester yields and monoester weight proportions of the examples and the comparative examples.
FIG. 11 is a table showing results of examples and comparative examples.
FIG. 12 is a graph showing time-series changes in monoester yields and monoester weight proportions of the examples and the comparative examples.

### Description of Embodiment

Hereinafter, a method for producing a sucrose fatty acid ester will be described, wherein the method includes: a step of producing a fatty acid alkali metal salt by mixing and heating a fatty acid ester and a solution in which an alkali metal salt is dissolved in solvent; a step of preparing an aqueous solution containing sucrose and a basic catalyst; and a step of producing a sucrose fatty acid ester, by mixing the aqueous solution obtained in the aforementioned step, the fatty acid alkali metal salt produced in the step of producing a fatty acid alkali metal salt, and a fatty acid ester, and heating the mixture with stirring under reduced pressure. The step of producing a sucrose fatty acid ester has an earlier step of removing water from the mixture, and a subsequent step of performing transesterification after the earlier step. In the subsequent step, heating is performed through microwave irradiation such that the temperature of the mixture is below the sucrose decomposition temperature.

The fatty acid ester used in the step of producing a fatty acid alkali metal salt may be, for example, a fatty acid alkylester, or may be a fatty acid polyhydric alcohol ester. There is no particular limitation on the constituent fatty acid of the fatty acid ester, and examples thereof include a short-chain fatty acid with 7 or less carbon atoms, a medium-chain fatty acid with 8 to 10 carbon atoms, and a long-chain fatty acid with 12 or more carbon atoms. The constituent fatty acid may be, for example, a fatty acid with 8 to 24 carbon atoms, may be a fatty acid with 8 to 22 carbon atoms, may be a fatty acid with 8 to 20 carbon atoms, or may be a fatty acid with 8 to 18 carbon atoms. The fatty acid may be a saturated fatty acid, or may be an unsaturated fatty acid. The constituent fatty acid of the fatty acid ester may be, for example, a fatty acid ester whose constituent fatty acid includes at least one selected from the group consisting of saturated fatty acids with 8 to 12 carbon atoms and saturated and unsaturated fatty acids with 14 to 24 carbon atoms. The unsaturated fatty acid may be a mono-unsaturated fatty acid (monoenoic fatty acid), or may be a poly-unsaturated fatty acid (polyenoic fatty acid). Examples of the fatty acid include caprylic acid, capric acid, lauric acid, myristic acid, myristoleic acid, palmitic acid, palmitoleic acid, stearic acid, oleic acid, linoleic acid, linolenic acid, eleostearic acid, arachidic acid, mead acid, arachidonic acid, behenic acid, lignoceric acid, nervonic acid, erucic acid, and the like.

The alkyl group in the fatty acid alkylester may be, for example, a linear or branched alkyl group with 1 to 5 carbon atoms. Examples of the alkyl group with 1 to 5 carbon atoms include a methyl group, an ethyl group, an *n*-propyl group, an isopropyl group, a cyclopropyl group, an *n*-butyl group, an isobutyl group, a *sec*-butyl group, a *t*-butyl group, a cyclobutyl group, a pentyl group, an isopentyl group, a neopentyl group, and a cyclopentyl group. The alkyl group is preferably a methyl group or an ethyl group, for example.

The polyhydric alcohol in the fatty acid polyhydric alcohol ester may be, for example, an alcohol with 2 to 6 hydroxyl groups. Examples of the polyhydric alcohol with 2 to 6 hydroxyl groups include glycol (ethylene glycol, propylene glycol, butylene glycol, etc.), glycerol (glycerin), pentaerythritol, and sorbitol.

Accordingly, there is no particular limitation on the fatty acid ester, and, for example, it may be either a fatty acid alkylester such as methyl caprylate, methyl caprate, methyl laurate, methyl myristate, methyl palmitate, methyl palmitoleate, methyl stearate, methyl oleate, methyl linoleate, ethyl caprylate, ethyl caprate, ethyl laurate, ethyl myristate, ethyl palmitate, ethyl palmitoleate, ethyl stearate, ethyl oleate, or ethyl linoleate, or a fatty acid polyhydric alcohol ester such as ethylene glycol fatty acid ester, propylene glycol fatty acid ester, butylene glycol fatty acid ester, glycerin fatty acid ester, pentaerythritol fatty acid ester, or sorbitol fatty acid ester. These fatty acid esters may be used alone or as a mixture of multiple types, but are preferably used alone in order to produce one type of sucrose fatty acid ester.

There is no particular limitation on the amount of fatty acid ester used in the step of producing a fatty acid alkali metal salt, and, if the fatty acid ester used to produce a fatty acid alkali metal salt is the same as the fatty acid ester used to produce a sucrose fatty acid ester and production of a sucrose fatty acid ester is performed using a fatty acid ester that has not been reacted in production of a fatty acid alkali metal salt, since the conversion percentage in the production of a fatty acid alkali metal salt is substantially 100%, the amount of fatty acid ester used in the step of producing a fatty acid alkali metal salt may be the same as or more than an amount that is obtained by adding the amount of fatty acid ester necessary to produce a desired fatty acid alkali metal salt and the amount of fatty acid ester necessary to produce a sucrose fatty acid ester. Meanwhile, if a fatty acid ester is added later, the amount of fatty acid ester used in the step of producing a fatty acid alkali metal salt may be the same as or more than the equivalent amount of desired fatty acid alkali metal salt.

The alkali metal salt is a salt of an alkali metal such as lithium, sodium, potassium, or the like. The salt may be, for example, hydroxide, carbonate, hydrogen carbonate, hydride, or alkoxide. There is no particular limitation on the alkali metal salt, and examples thereof include potassium hydroxide, potassium carbonate, potassium hydrogen carbonate, methoxypotassium (potassium methoxide), sodium hydroxide, sodium carbonate, sodium hydrogen carbonate, and sodium methoxide. These alkali metal salts may be used alone or as a mixture of multiple types. The amount of alkali metal salt used is preferably an amount that allows a necessary amount of fatty acid alkali metal salt to be produced in the step of producing a fatty acid ester, and may be, for example, an amount that is the same as the equivalent amount of desired fatty acid alkali metal salt. As the alkali metal salt, potassium hydroxide or sodium hydroxide is preferably used.

There is no particular limitation on the solvent as long as an alkali metal salt is soluble therein, but it may be, for example, at least one selected from the group consisting of alcohol, ketone, and water. There is no particular limitation on the alcohol, and examples thereof include an alcohol with 1 to 5 carbon atoms. Examples of the alcohol with 1 to 5 carbon atoms include methanol, ethanol, propanol (1-propanol), isopropanol (2-propanol), *n*-butanol, isobutanol, *sec*-butanol, *tert*-butanol, *n*-amyl alcohol, *sec*-amyl alcohol, 3-pentanol, 2-methyl-1-butanol, isoamyl alcohol, *tert*-amyl alcohol, 3-methyl-2-butanol, and neopentyl alcohol. These alcohols may be used alone or as a mixture of multiple types. Examples of the ketone with 3 to 5 carbon atoms include acetone, methyl ethyl ketone, and diethyl ketone. These ketones may be used alone or as a mixture of multiple types. The solvent is preferably methanol or ethanol, because they can be easily removed later. Also, the solvent is preferably the same as the solvent obtained by reaction between a fatty acid ester and an alkali metal salt. For example, if a fatty acid alkylester having a methyl group or an ethyl group and an alkali metal hydroxide are reacted, methanol or ethanol is preferably used as the solvent. Since this solvent is irrelevant to the reaction, the amount of solvent used may be freely selected in the range where an alkali metal salt is dissolved.

When dissolving the alkali metal salt in the solvent, stirring may or may not be performed. Furthermore, when dissolving the alkali metal salt, heating may or may not be performed.

In the mixed liquid in which a fatty acid ester and an alkali metal salt solution are mixed, the fatty acid ester is preferably in a liquid form. In the mixed liquid, the fatty acid ester may be, for example, melted, or may be dissolved. If the fatty acid ester is solid at room temperature, the fatty acid ester melted or dissolved in solvent in advance may be mixed with the alkali metal salt solution, or the fatty acid ester in a solid form may be mixed with the alkali metal salt solution and then melted or dissolved. In the former case, for example, the fatty acid ester in a solid form may be melted by being heated at a temperature that is at or above the melting point, or the fatty acid ester in a solid form may be dissolved in organic solvent such as non-polar solvent such as alcohol, ketone, hexane, or heptane. When dissolving the fatty acid ester in a solid form in the organic solvent, heating may or may not be performed. Furthermore, heating performed when melting the fatty acid ester in a solid form or dissolving it in solvent may or may not be performed through microwave irradiation.

It is possible to produce a fatty acid alkali metal salt by mixing and heating a fatty acid ester and a solution in which an alkali metal salt is dissolved in solvent. The heating is performed preferably using microwaves, but there is no limitation to this. At the time of heating, reflux is preferably performed. There is no particular limitation on the heating temperature, but it is preferably in the range of 30°C to 200°C, and more preferably 60°C or more. Also, there is no particular limitation on the heating time, but it is preferably in the range of 5 minutes to 24 hours, more preferably in the range of 20 minutes to 2 hours, and even more preferably in the range of 30 minutes to 1 hour. Furthermore, the reaction is caused to occur preferably under reduced pressure. At the start of the heating, the pressure may be reduced pressure, or may be ordinary pressure. If the pressure is ordinary pressure at the start of the heating, the reduction of pressure may be started together with the heating. The pressure is reduced preferably to 1 to 90 kPa, more preferably to 1 to 10 kPa, and even more preferably to 1 to 4 kPa. Examples of the alkali metal of the fatty acid alkali metal salt produced through the reaction are as described above, and examples of the fatty acid are also as described above. Accordingly, examples of the fatty acid alkali metal salt produced include potassium caprylate, potassium caprate, potassium laurate, potassium myristate, potassium palmitate, potassium palmitoleate, potassium stearate, potassium oleate, potassium linoleate, sodium caprylate, sodium caprate, sodium laurate, sodium myristate, sodium palmitate, sodium palmitoleate, sodium stearate, sodium oleate, and sodium linoleate. The fatty acid alkali metal salt produced in this step is not dissolved in the solvent, and thus the fatty acid alkali metal salt can be obtained by removing the solvent. The solvent can be removed by, for example, keeping the mixture in a heated state or under reduced pressure. The fatty acid alkali metal salt may or may not contain an unreacted fatty acid ester. Note that, after the reaction ends, the temperature is preferably lowered. There is no particular limitation on the temperature that has been lowered, but, for example, it is preferably a temperature that is below 100°C, or a temperature that is at or above the melting point of the unreacted fatty acid ester.

There is no particular limitation on the basic catalyst as long as it is a catalyst having a basicity, but it may be, for example, an alkali metal salt, an alkaline-earth metal salt, or a metal salt. The alkali metal salt is, for example, a salt of an alkali metal such as lithium, sodium, potassium, or the like. The alkaline-earth metal salt is, for example, a salt of an alkaline-earth metal such as beryllium, magnesium, calcium, or the like. The metal salt is, for example, a salt of a metal such as manganese, zinc, copper, nickel, or the like. As described above, the salt may be, for example, hydroxide, carbonate, hydrogen carbonate, hydride, or alkoxide. There is no particular limitation on the alkali metal salt, and examples thereof include potassium hydroxide, potassium carbonate, potassium hydrogen carbonate, methoxypotassium, sodium hydroxide, sodium carbonate, sodium hydrogen carbonate, and sodium methoxide. There is no particular limitation on the alkaline-earth metal salt, and examples thereof include magnesium hydroxide, magnesium carbonate, magnesium hydrogen carbonate, magnesium methoxide, calcium hydroxide, calcium carbonate, calcium hydrogen carbonate, and calcium methoxide. There is no particular limitation on the metal salt, and examples thereof include manganese hydroxide and zinc hydroxide. These basic catalysts may be used alone or as a mixture of multiple types. Such a basic catalyst is at least partially dissolved in water. Accordingly, the basic catalyst may be referred to as a water-soluble catalyst. Note that the basic catalyst is preferably completely dissolved in water. Furthermore, since the basic catalyst is used in esterification of a hydroxyl group of sucrose, it may be referred to as an esterification catalyst. The basic catalyst is preferably a salt of an alkali metal that is the same as the alkali metal of the fatty acid alkali metal salt. For example, if the alkali metal of the fatty acid alkali metal salt is potassium or sodium, the basic catalyst is preferably potassium hydroxide or sodium hydroxide.

There is no limitation on the amount of water in the step of preparing an aqueous solution containing sucrose and a basic catalyst, but the amount of water is preferably the minimum amount in the range where sucrose is dissolved because the water has to be removed later. The amount of basic catalyst is preferably in the range of 0.01 to 20% by weight, and more preferably in the range of 0.1 to 5% by weight, with respect to the sucrose. Furthermore, in this step, for example, stirring such as rotation stirring or swing stirring may be performed in order to dissolve the sucrose and the basic catalyst in water. In this step, heating may or may not be performed. There is no limitation on the order in which the sucrose and the basic catalyst are dissolved in water.

Examples of the fatty acid ester used in the step of producing a sucrose fatty acid ester are as described above. The fatty acid ester may be the same as or different from the fatty acid ester used in the step of producing a fatty acid alkali metal salt. In the former case, as described above, an unreacted fatty acid ester that has not been used in the reaction in the step of producing a fatty acid alkali metal salt may be used in the step of producing a sucrose fatty acid ester, or the same fatty acid ester may be newly added. If the fatty acid ester is newly added and the fatty acid ester that is to be added is solid at room temperature, the fatty acid ester may be melted and then added, or may be added and then melted. In either case, the fatty acid ester is preferably melted in production of a sucrose fatty acid ester. The fatty acid ester used to produce a sucrose fatty acid ester being the same as the fatty acid ester used to produce a fatty acid alkali metal salt is advantageous in that the reusability is good, because, even if an excess amount of fatty acid ester is used with respect to sucrose, another type of fatty acid ester is not mixed in during collecting the fatty acid ester. The fatty acid alkali metal salt can be used as an emulsifier in a mixture of an aqueous solution of sucrose and a fatty acid ester.

Examples of the fatty acid alkali metal salt used in the step of producing a sucrose fatty acid ester are as described above, and these fatty acid alkali metal salts may be used alone or as a mixture of multiple types. There is no particular limitation on the amount of fatty acid alkali metal salt used, but, for example, it is preferably 1 to 50% by weight with respect to the entire mixture, and more preferably 10 to 30% by weight if the fatty acid ester used to produce a sucrose fatty acid ester is a fatty acid alkylester.

In the step of producing a sucrose fatty acid ester, an aqueous solution in which sucrose is dissolved in water in the presence of a basic catalyst, the fatty acid alkali metal salt produced in the earlier step, and a fatty acid ester are mixed and heated with stirring under reduced pressure, so that a sucrose fatty acid ester is produced. Regarding the mixing, there is no limitation on the order of the mixing as long as the aqueous solution, the fatty acid alkali metal salt, and the fatty acid ester are eventually mixed. It is sufficient that the fatty acid ester is eventually melted after the mixing. Accordingly, the fatty acid ester may be melted at the time of mixing, or may not be melted at the time of mixing and melted after the mixing. In the former case, if the fatty acid ester is solid at room temperature, for example, the fatty acid ester may be melted by being heated. The mixed liquid preferably does not contain organic solvent. Furthermore, the amount of fatty acid ester that is mixed with sucrose in this step is preferably in the range of 1 to 20 equivalents with respect to one equivalent of sucrose. In this manner, the amount of fatty acid ester used is preferably the same as or more than the equivalent amount of sucrose. The stirring performed in this step may be, for example, rotation stirring, swing stirring, or ultrasonic stirring, or may be any combination of two or more. The stirring may be continuously performed, or may be intermittently performed. Note that the mixture in which the aqueous solution of sucrose, the fatty acid alkali metal salt, and the fatty acid ester are mixed is a fluid having high viscosity. Accordingly, this mixture may be considered as a mixed liquid having high viscosity.

The step of producing a sucrose fatty acid ester has an earlier step of removing water from the mixture, and a subsequent step of performing transesterification in a state where the water has been removed after the earlier step. In the earlier step, it is preferable that stirring is sufficiently performed in order to remove water in a state where a dispersoid that is the aqueous solution of sucrose is uniformly dispersed. Also in the subsequent step, it is preferable that stirring is sufficiently performed in order to facilitate esterification between the sucrose and the fatty acid ester.

The heating in the earlier step in the step of producing a sucrose fatty acid ester may or may not be performed through microwave irradiation. The heating in the subsequent step in the step of producing a sucrose fatty acid ester is performed through microwave irradiation. There is no particular limitation on the frequency of the microwaves, but it may be, for example, 2.45 GHz, 5.8 GHz, 24 GHz, 915 MHz, or another frequency in the range of 300 MHz to 300 GHz. Furthermore, microwaves having the same frequency may be irradiated, or microwaves having multiple frequencies may be irradiated. In the latter case, for example, microwaves having multiple frequencies may be simultaneously irradiated, or may be irradiated at different times. If microwaves having multiple frequencies are irradiated at different times, for example, microwaves having a frequency that are easily absorbed by the raw material may be irradiated at the start of the reaction, and microwaves having a frequency that are easily absorbed by the product may be irradiated when the reaction has progressed. Furthermore, for example, microwaves having multiple frequencies may be irradiated at the same position, or may be irradiated at different positions. If microwaves having multiple frequencies are irradiated at different positions, for example, microwaves having a frequency that are easily absorbed by the raw material may be irradiated at an upstream position in a flow-type reactor, that is, at a position where the proportion of the raw material is larger than that of the product, and microwaves having a frequency that are easily absorbed by the product may be irradiated at a downstream position in the reactor, that is, at a position where the proportion of the product is larger than that of the raw material. The microwaves may be continuously irradiated, or may be intermittently irradiated such that irradiation and halt are repeated. With microwave irradiation, the temperature of a material irradiated with the microwaves increases. The intensity of the microwave irradiation may be adjusted such that the temperature is kept constant, the intensity of the microwave irradiation may be kept constant such that the temperature changes, or the intensity of the microwave irradiation may be frequently changed. The temperature of the mixture that is irradiated with the microwaves may be measured, for example, using a known thermometer such as a thermocouple-type thermometer or an infrared fiber-type thermometer. The measured temperature may be used to control the output (intensity) of the microwaves. The microwave may be irradiated in a single mode or a multi-mode. A microwave-absorbing catalyst may or may not be added to the mixture that is irradiated with the microwaves. If the sucrose fatty acid ester that is produced is to be used in foods or drugs, and a catalyst has to be completely removed after the reaction, no catalyst is preferably used.

The temperature in the earlier step, that is, the step of removing water from the mixture is preferably below the reaction temperature in the subsequent step, that is, the step of performing transesterification. The reason for this is that, if the temperature in the earlier step is at or above the reaction temperature in the subsequent step of performing transesterification, transesterification starts in the earlier step and the yield of sucrose fatty acid ester in the subsequent step is lowered. On the other hand, if the temperature in the earlier step is too low, water cannot be sufficiently removed, and the reaction in the subsequent step is inhibited. Accordingly, the temperature of the mixture in the earlier step is preferably above room temperature and below the reaction temperature in the subsequent step. For example, the heating in the earlier step is performed at a temperature of preferably in the range of 40°C to 80°C, and more preferably in the range of 50°C to 80°C. Furthermore, the time of the earlier step is preferably in the range of 5 minutes to 24 hours, more preferably in the range of 20 minutes to 2 hours, and even more preferably in the range of 30 minutes to 1 hour. The water is removed preferably such that the amount of remaining water becomes 0.1 to 2% by weight with respect to the entire mixture.

In the subsequent step, the heating is performed such that the temperature of the mixture is below the sucrose decomposition temperature. The reason for this is that, if the heating is performed such that the temperature is at or above the sucrose decomposition temperature, odoring (so-called caramel odor) occurs, and thus the quality of the sucrose fatty acid ester deteriorates. Note that the sucrose decomposition temperature is a temperature at which sucrose starts to decompose. The decomposition temperature at which sucrose heated in a short time causes odoring is approximately 135°C or more. In the subsequent step, for example, the heating is performed preferably in the range of a temperature that is 25°C lower than the sucrose decomposition temperature to a temperature that is below the sucrose decomposition temperature, and more preferably in the range of a temperature that is 10°C lower than the sucrose decomposition temperature to a temperature that is below the sucrose decomposition temperature. The reason for this is that, if the reaction temperature in the subsequent step is closer to the sucrose decomposition temperature, a higher monoester yield can be realized in a shorter time. In the subsequent step, the mixture is heated preferably in the range of 100°C to 135°C, more preferably in the range of 110°C to 133°C, and even more preferably in the range of 110°C to 130°C. Furthermore, in the subsequent step, the mixture may be heated at 120°C or more. In the subsequent step, the mixture may be heated with microwaves such that its measured temperature is "sucrose decomposition temperature - α (°C)", where α is a positive real number. It is preferable that α is a small value, but the value of α may be selected according to the responsiveness of feedback of the temperature control with microwaves. Specifically, the value of α may be made smaller if the responsiveness is high, and may be made larger if the responsiveness is low. The reaction time (heating time) of the subsequent step is preferably 5 minutes to 6 hours. The lower limit of the reaction time may be, for example, 30 minutes or more, or 1 hour or more. The upper limit of the reaction time may be, for example, 6 hours or less, 5 hours or less, 4 hours or less, 3.5 hours or less, or 3 hours or less. Since a longer time of the subsequent step makes the monoester selectivity lower, a shorter time of the subsequent step is more preferable. On the other hand, in order to realize a high monoester yield, the time of the subsequent step preferably has a length with which the monoester yield reaches a peak. The temperature may be constant, or may be changed. In the latter case, in the subsequent step of performing transesterification, for example, the heating may be performed such that the temperature of the mixture increases. The temperature may be increased, for example, through linear temperature change, stepwise temperature change, or other temperature increase. If the heating is performed in the subsequent step such that the temperature of the mixture increases, the temperature preferably monotonically increases throughout the period of the subsequent step, but it is sufficient that the temperature increases as a whole in the period of the subsequent step, and there may be a period in which the temperature decreases in a microscopic point of view. It is conceivable that there is such a period, for example, if microwaves are intermittently irradiated. Even if the temperature changes in the subsequent step, it is important that the temperature does not reach the sucrose decomposition temperature or more. The produced sucrose fatty acid ester preferably has a high monoester content. For example, in the produced sucrose fatty acid ester, the monoester weight proportion is preferably larger than the weight proportion of the sucrose fatty acid ester in which two or more hydroxyl groups of sucrose have been esterified by the fatty acid ester. For example, in the produced sucrose fatty acid ester, the monoester weight proportion is preferably 60% by weight or more, more preferably 70% by weight or more, and even more preferably 80% by weight or more. It is known that a monoester of a sucrose fatty acid ester has antimicrobial properties, and, furthermore, a sucrose fatty acid ester having a higher monoester content is more likely to be dissolved in water, and thus, if a sucrose fatty acid ester is used in, for example, foods or drinks, a high monoester content is required. Furthermore, for use in drugs and the like, a highly pure monoester may be required, and, in such a case, a highly pure monoester is obtained preferably from a sucrose fatty acid ester having a high monoester content, and thus the produced sucrose fatty acid ester preferably has a high monoester proportion. As will be described in the following examples, for example, if the reaction temperature in the subsequent step in the case of producing a sucrose palmitic acid ester is 120°C, the heating time is preferably 3 hours or less, and more preferably 2.5 hours or less. Furthermore, for example, if the reaction temperature in the subsequent step in the case of producing a sucrose stearic acid ester is 130°C, the heating time is preferably 4 hours or less, and more preferably 3.5 hours or less. Furthermore, it is preferable that the yield of the produced monoester of the sucrose fatty acid ester is high. According to the present invention, such a high monoester selectivity and high monoester yield can be realized by performing heating with microwaves.

The pressures in the earlier and subsequent steps are preferably 1 to 90 kPa, and more preferably 1 to 10 kPa. The pressures in the earlier step and in the subsequent step may be the same or different from each other. In the latter case, for example, the pressure in the subsequent step may be higher than the pressure in the earlier step. The degree of the reduction in the pressure may be in the range where the raw material (e.g., fatty acid ester, etc.) is not vaporized.

In the step of producing a sucrose fatty acid ester, transesterification is performed, so that a sucrose fatty acid ester is produced. There is no particular limitation on the sucrose fatty acid ester, and examples thereof include a sucrose caprylic acid ester, a sucrose capric acid ester, a sucrose lauric acid ester, a sucrose myristic acid ester, a sucrose myristoleic acid ester, a sucrose palmitic acid ester, a sucrose palmitoleic acid ester, a sucrose stearic acid ester, a sucrose oleic acid ester, a sucrose linoleic acid ester, a sucrose linolenic acid ester, a sucrose eleostearic acid ester, a sucrose arachidic acid ester, a sucrose mead acid ester, a sucrose arachidonic acid ester, a sucrose behenic acid ester, a sucrose lignoceric acid ester, a sucrose nervonic acid ester, and a sucrose erucic acid ester, and the like. In the subsequent step of producing a sucrose fatty acid ester, the mixture may be cooled down after the transesterification, in order to prevent reactions that change monoester into a diester.

As the method for extracting the produced sucrose fatty acid ester, ordinary methods may be used. For example, if water and organic solvent (e.g., methyl ethyl ketone, water-insoluble alcohol, etc.) are added to and stirred with the product in the step of producing a sucrose fatty acid ester, the mixture is separated into two layers consisting of a water layer and an organic solvent layer. The organic solvent contains an unreacted fatty acid ester and a sucrose fatty acid ester. The water contains an unreacted sucrose and a fatty acid alkali metal salt. The sucrose fatty acid ester can be extracted, for example, by crystallizing and extracting the sucrose fatty acid ester from the organic solvent.

As described above, with the method for producing a sucrose fatty acid ester according to the present invention, it is possible to produce a sucrose fatty acid ester with no odoring, by heating the mixture such that the temperature is below the decomposition in the subsequent step of producing a sucrose fatty acid ester. Furthermore, it is possible to obtain a sucrose fatty acid ester having a high monoester selectivity and a high monoester yield in a short time, by performing heating with microwaves. Furthermore, since no toxic organic solvent is used in the step of producing a sucrose fatty acid ester, it is possible to produce a sucrose fatty acid ester that is preferable for use in foods, drugs, and cosmetics.

In the description above, the case was described in which the method for producing a sucrose fatty acid ester includes a step of producing a fatty acid alkali metal salt, but there is no limitation to this. If a fatty acid alkali metal salt is prepared in advance, the fatty acid alkali metal salt may be used in the step of producing a sucrose fatty acid ester. Furthermore, the fatty acid alkali metal salt may be produced by a method different from that described above.

### Examples and Comparative Examples

Hereinafter, the present invention will be described in detail by way of examples, but these example are merely examples and the present invention is not limited to these examples.

### 1. Method for Producing Sucrose Palmitic Acid Ester

### Example 1

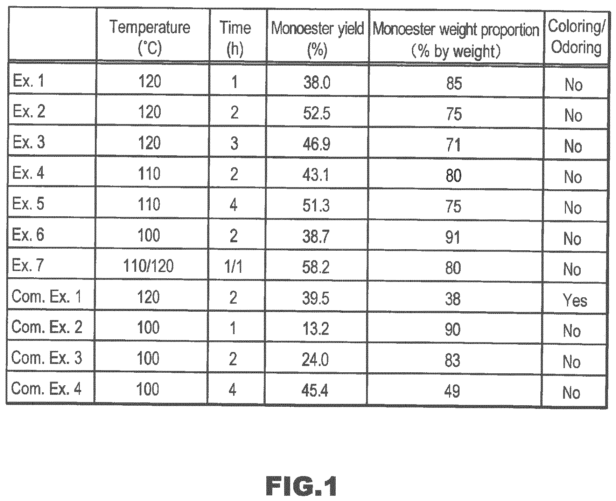

In this example, 98 g of methyl palmitate melted by being heated to 60°C and a solution in which 3.8 g of potassium hydroxide was dissolved in 30 ml of methanol were added to a three-necked flask. After the three-necked flask was placed in a microwave reactor (µReactor Ex, manufactured by Shikoku Instrumentation Co., Ltd.) provided with a stirrer and a thermometer, the content was irradiated with microwaves at 2.45 GHz, heated to 100°C with stirring, and refluxed for 10 minutes. Then, the temperature was kept at 100°C while reducing the pressure to 4 kPa, so that potassium palmitate was produced. After methanol was sufficiently removed by maintaining this state, the content was cooled down to 60°C. The content was a mixture of 80 g of methyl palmitate and 20 g of potassium palmitate. An aqueous solution in which 12 g of sucrose and 0.5 g of potassium hydroxide were dissolved in 12 g of water was added to the three-necked flask. While keeping the mixture in the three-necked flask at 60°C, the pressure was reduced to 4 kPa with stirring. This state was maintained for 30 minutes, so that water was evaporated. Then, while maintaining the stirring and the reduced pressure, microwaves were irradiated to increase the temperature to 120°C, and transesterification was performed for 1 hour, so that a sucrose palmitic acid ester was produced. Note that there was neither coloring nor odoring from the start to the end of the reaction. That is to say, during the reaction, the temperature was kept below a temperature at which sucrose starts to decompose. After the reaction ended, acetone was used to obtain a sucrose palmitic acid ester from the reacted material. The yield and the monoester proportion of the sucrose palmitic acid ester were analyzed using high performance liquid chromatography and gas chromatography. The results are as shown in FIG. 1.

### Example 2

A sucrose palmitic acid ester was produced as in Example 1, except that the transesterification time was changed from 1 hour to 2 hours. Also in this case, there was neither coloring nor odoring from the start to the end of the reaction. The results are as shown in FIG. 1.

### Example 3

A sucrose palmitic acid ester was produced as in Example 1, except that the transesterification time was changed from 1 hour to 3 hours. Also in this case, there was neither coloring nor odoring from the start to the end of the reaction. The results are as shown in FIG. 1.

### Example 4

A sucrose palmitic acid ester was produced as in Example 2, except that the temperature in the transesterification was changed from 120°C to 110°C. Also in this case, there was neither coloring nor odoring from the start to the end of the reaction. The results are as shown in FIG. 1.

### Example 5

A sucrose palmitic acid ester was produced as in Example 4, except that the transesterification time was changed from 2 hours to 4 hours. Also in this case, there was neither coloring nor odoring from the start to the end of the reaction. The results are as shown in FIG. 1.

### Example 6

A sucrose palmitic acid ester was produced as in Example 2, except that the temperature in the transesterification was changed from 120°C to 100°C. Also in this case, there was neither coloring nor odoring from the start to the end of the reaction. The results are as shown in FIG. 1.

### Example 7

A sucrose palmitic acid ester was produced as in Example 1, except that the temperature in the transesterification was set to 110°C for 1 hour in the first half and to 120°C for 1 hour in the second half so that the transesterification was performed for 2 hours in total. The temperature was increased from 110°C to 120°C swiftly, and, also at that time, the reduced pressure was maintained. Also in this case, there was neither coloring nor odoring from the start to the end of the reaction. The results are as shown in FIG. 1.

### Comparative Example 1

A sucrose palmitic acid ester was produced as in Example 2, except that the heating with microwave irradiation was changed to heating with an oil bath (conventional heating). In this case, in the transesterification, the reacted liquid was colored dark brown, and there was a smell of burnt sucrose. The results are as shown in FIG. 1.

Also in the case where the temperature in the transesterification was changed from 120°C to 110°C, the reacted liquid was colored dark brown as in Comparative Example 1.

### Comparative Example 2

A sucrose palmitic acid ester was produced as in Comparative Example 1, except that the temperature in the transesterification was changed from 120°C to 100°C and the transesterification time was changed from 2 hours to 1 hour. In this case, there was neither coloring nor odoring from the start to the end of the reaction. The results are as shown in FIG. 1.

### Comparative Example 3

A sucrose palmitic acid ester was produced as in Comparative Example 2, except that the transesterification time was changed from 1 hour to 2 hours. Also in this case, there was neither coloring nor odoring from the start to the end of the reaction. The results are as shown in FIG. 1.

### Comparative Example 4

A sucrose palmitic acid ester was produced as in Comparative Example 2, except that the transesterification time was changed from 1 hour to 4 hours. Also in this case, there was neither coloring nor odoring from the start to the end of the reaction. The results are as shown in FIG. 1.

In order to prevent coloring and odoring in conventional heating, the temperature has to be lowered. Furthermore, if a sucrose fatty acid ester is produced at a lower temperature in the conventional heating, as can be seen from the results of Comparative Examples 2 to 4, the monoester weight proportion is lower if the monoester yield is higher, and the monoester yield is lower if the monoester weight proportion is higher. On the other hand, in the case of Examples 1 to 7 where heating was performed with microwaves, both a high monoester yield and a high monoester weight proportion were realized compared with Comparative Examples 2 to 4. In particular, a yield that was at least 1.5 times as high as that of Comparative Examples 2 to 4 was realized as well as a monoester weight proportion of 70% by weight or more. In Example 2, such a high monoester yield and high monoester weight proportion were realized in a short time.

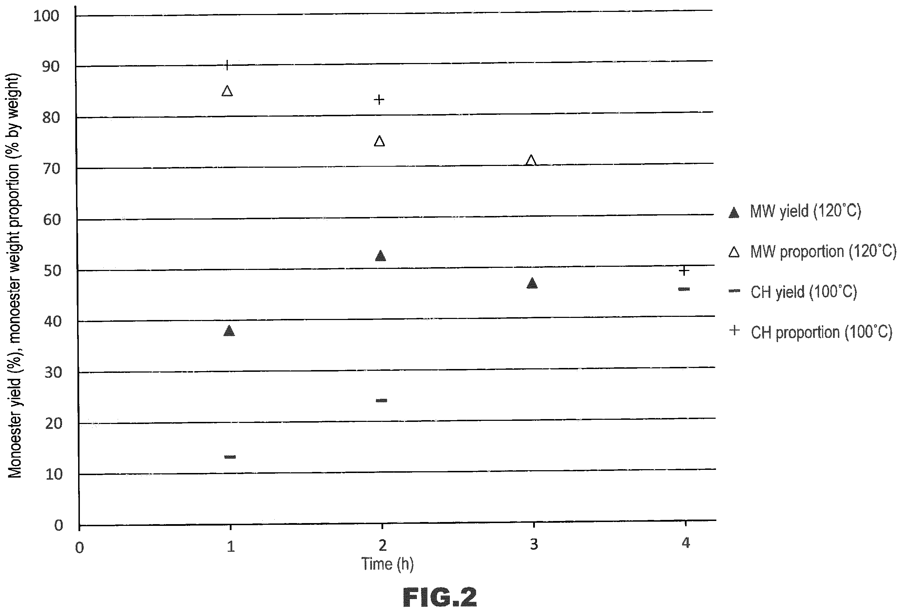

FIG. 2 is a graph showing time-series changes in the monoester yields and the monoester weight proportions of Examples 1 to 3 and Comparative Examples 2 to 4. In production of a sucrose fatty acid ester, the reaction progresses such that a monoester is first produced, a diester is produced from the monoester, and a triester is produced from the diester, and thus, as shown in the changes in the yields in Examples 1 to 3, a monoester yield gradually increases in accordance with the elapse of time, and takes a downward turn at a certain point during the reaction. Furthermore, since a diester and the like are produced in accordance with the elapse of time, a monoester proportion gradually decreases from 100%.

In FIG. 2, it is seen that the time of the time-series change in the monoester yield in microwave heating (MW) was shortened to be smaller than 1/2 the time of the time-series change in the monoester yield in conventional heating (CH). The reason for this is that, assuming that the change in the yield up to 4 hours in the conventional heating was a monotonic increase, for example, the microwave heating time corresponding to the yield at 2 hours in the conventional heating was shorter than 1 hour, and the microwave heating time corresponding to the yield at 4 hours in the conventional heating was also shorter than 2 hours. On the other hand, it is seen that the time of the time-series change in the monoester proportion in the microwave heating was not shortened to be 1/2 the time of the time-series change in the monoester proportion in the conventional heating. The reason for this is that the monoester weight proportion (83%) in Comparative Example 3 was smaller than the monoester weight proportion (85%) in Example 1. In this manner, it is seen that, in the reaction that sequentially esterifies multiple hydroxyl groups of sucrose, if the conventional heating is changed to the microwave heating, the monoester yield is shortened to be shorter in the time direction than the monoester proportion. Due to such a difference, the microwave heating can simultaneously realize both a high monoester yield and a high monoester selectivity in the range of a short reaction time (e.g., the range of a reaction time of 4 hours or shorter, etc.).

Furthermore, as shown in Example 7, it is seen that it is possible to realize both a higher monoester yield and a higher monoester selectivity by increasing the reaction temperature in a stepwise manner in the transesterification. Accordingly, it seems that it is possible to realize both a higher monoester yield and a higher monoester selectivity by performing the heating such that the temperature of the mixture increases in the transesterification.

Referring to FIG. 2, it is seen that the monoester yield in the microwave heating at 120°C (Examples 1 to 3) had a peak with a heating time of approximately 2 hours. Accordingly, the transesterification time (i.e., the heating time of the subsequent step) may be set to a time at which the monoester yield reaches a peak. Note that the number of points in time at which the monoester yield reaches a peak is to be theoretically one, but it is actually difficult to specify the point in time. Accordingly, the time at which the monoester yield reaches a peak may be considered as a period of time having a width. For example, the transesterification time at which the monoester yield reached a peak in the microwave heating at 120°C (Examples 1 to 3) may be considered to be in the range of 1.5 hours to 2.5 hours.

Furthermore, a comparison between the results of Examples 2, 4, and 6 shows that, in the range of a temperature that is below the sucrose decomposition temperature, a higher monoester yield can be realized at a higher reaction temperature. Accordingly, it is seen that the reaction is caused to occur preferably at a reaction temperature that is closer to the sucrose decomposition temperature.

### 2. Method for Producing Sucrose Stearic Acid Ester

### Example 8

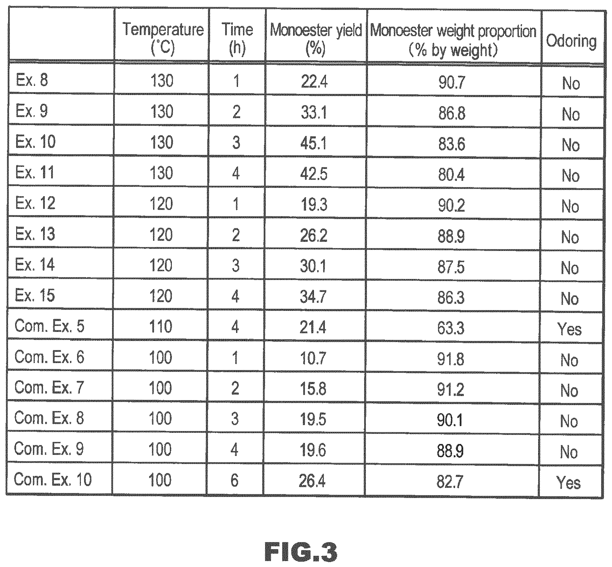

In this example, 108.6 g of methyl stearate melted by being heated to 60°C and a solution in which 3.8 g of potassium hydroxide was dissolved in 30 ml of methanol were added to a three-necked flask. After the three-necked flask was placed in a microwave reactor (µReactor Ex, manufactured by Shikoku Instrumentation Co., Ltd.) provided with a stirrer and a thermometer, the content was irradiated with microwaves at 2.45 GHz, heated to 100°C with stirring, and refluxed for 10 minutes. Then, the temperature was kept at 100°C while reducing the pressure to 4 kPa, so that potassium stearate was produced. After methanol was sufficiently removed by maintaining this state, the content was cooled down to 80°C. The content was a mixture of 88.3 g of methyl stearate and 21.9 g of potassium stearate. An aqueous solution in which 12 g of sucrose and 0.5 g of potassium hydroxide were dissolved in 8 g of water was added to the three-necked flask. While keeping the mixture in the three-necked flask at 80°C, the pressure was reduced to 4 kPa with stirring, so that water was evaporated. Then, while maintaining the stirring and the reduced pressure, microwaves were irradiated to increase the temperature to 130°C, and transesterification was performed for 1 hour, so that a sucrose stearic acid ester was produced. Note that there was no odoring from the start to the end of the reaction. That is to say, during the reaction, the temperature was kept below a temperature at which sucrose starts to decompose. After the reaction ended, acetone was used to obtain a sucrose stearic acid ester from the reacted material. The yield and the monoester proportion of the sucrose stearic acid ester were analyzed using high performance liquid chromatography and gas chromatography. The results are as shown in FIG. 3.

### Example 9

A sucrose stearic acid ester was produced as in Example 8, except that the transesterification time was changed from 1 hour to 2 hours. Also in this case, there was no odoring from the start to the end of the reaction. The results are as shown in FIG. 3.

### Example 10

A sucrose stearic acid ester was produced as in Example 8, except that the transesterification time was changed from 1 hour to 3 hours. Also in this case, there was no odoring from the start to the end of the reaction. The results are as shown in FIG. 3.

### Example 11

A sucrose stearic acid ester was produced as in Example 8, except that the transesterification time was changed from 1 hour to 4 hours. Also in this case, there was no odoring from the start to the end of the reaction. The results are as shown in FIG. 3.

### Example 12

A sucrose stearic acid ester was produced as in Example 8, except that the temperature in the transesterification was changed from 130°C to 120°C. Also in this case, there was no odoring from the start to the end of the reaction. The results are as shown in FIG. 3.

### Example 13

A sucrose stearic acid ester was produced as in Example 12, except that the transesterification time was changed from 1 hour to 2 hours. Also in this case, there was no odoring from the start to the end of the reaction. The results are as shown in FIG. 3.

### Example 14

A sucrose stearic acid ester was produced as in Example 12, except that the transesterification time was changed from 1 hour to 3 hours. Also in this case, there was no odoring from the start to the end of the reaction. The results are as shown in FIG. 3.

### Example 15

A sucrose stearic acid ester was produced as in Example 12, except that the transesterification time was changed from 1 hour to 4 hours. Also in this case, there was no odoring from the start to the end of the reaction. The results are as shown in FIG. 3.

### Comparative Example 5

A sucrose stearic acid ester was produced as in Example 11, except that the heating with microwave irradiation was changed to heating with an oil bath (conventional heating) and the temperature in the transesterification was changed from 130°C to 110°C. In this case, in the transesterification, there was a smell of burnt sucrose in the reacted liquid. The results are as shown in FIG. 3.

Furthermore, when the temperature in the transesterification was set to 130°C and to 120°C in the heating with an oil bath, the reacted liquid was burnt black, and there was a smell of burnt sucrose. Accordingly, if heating is performed at a temperature of 110°C or more in the conventional heating, it is difficult to produce a sucrose stearic acid ester with no odoring.

### Comparative Example 6

A sucrose stearic acid ester was produced as in Comparative Example 5, except that the temperature in the transesterification was changed from 110°C to 100°C and the transesterification time was changed from 4 hours to 1 hour. In this case, there was no odoring from the start to the end of the reaction. The results are as shown in FIG. 3.

### Comparative Example 7

A sucrose stearic acid ester was produced as in Comparative Example 6, except that the transesterification time was changed from 1 hour to 2 hours. Also in this case, there was no odoring from the start to the end of the reaction. The results are as shown in FIG. 3.

### Comparative Example 8

A sucrose stearic acid ester was produced as in Comparative Example 6, except that the transesterification time was changed from 1 hour to 3 hours. Also in this case, there was no odoring from the start to the end of the reaction. The results are as shown in FIG. 3.

### Comparative Example 9

A sucrose stearic acid ester was produced as in Comparative Example 6, except that the transesterification time was changed from 1 hour to 4 hours. Also in this case, there was no odoring from the start to the end of the reaction. The results are as shown in FIG. 3.

### Comparative Example 10

A sucrose stearic acid ester was produced as in Comparative Example 6, except that the transesterification time was changed from 1 hour to 6 hours. In this case, in the transesterification, there was a smell of burnt sucrose in the reacted liquid. The results are as shown in FIG. 3.

In order to prevent odoring in conventional heating, the temperature has to be lowered. Furthermore, if a sucrose stearic acid is produced at a lower temperature in the conventional heating, as can be seen from the results of Comparative Examples 6 to 10, the monoester weight proportion is lower if the monoester yield is higher, and the monoester yield is lower if the monoester weight proportion is higher. On the other hand, in the case of Examples 8 to 15 where heating was performed with microwaves, both a high monoester yield and a high monoester weight proportion were realized compared with Comparative Examples 6 to 9. For example, a comparison between the monoester yields in Example 10 and Comparative Example 10 having similar monoester weight proportions shows that, with the microwave heating, a yield that was 1.7 times as high as that of the conventional heating was realized. Furthermore, with the microwave heating, such a high yield was realized without causing odoring. In the conventional heating, time-series changes in the monoester yield were gradual, and, furthermore, heating for a long time caused odoring (see Comparative Example 10, for example), and thus a high monoester yield was not realized, whereas, in Examples 10, 11, 15, etc., yields higher than those in the comparative examples were realized. In particular in Example 10, a high monoester yield and a high monoester weight proportion were realized in a short time.

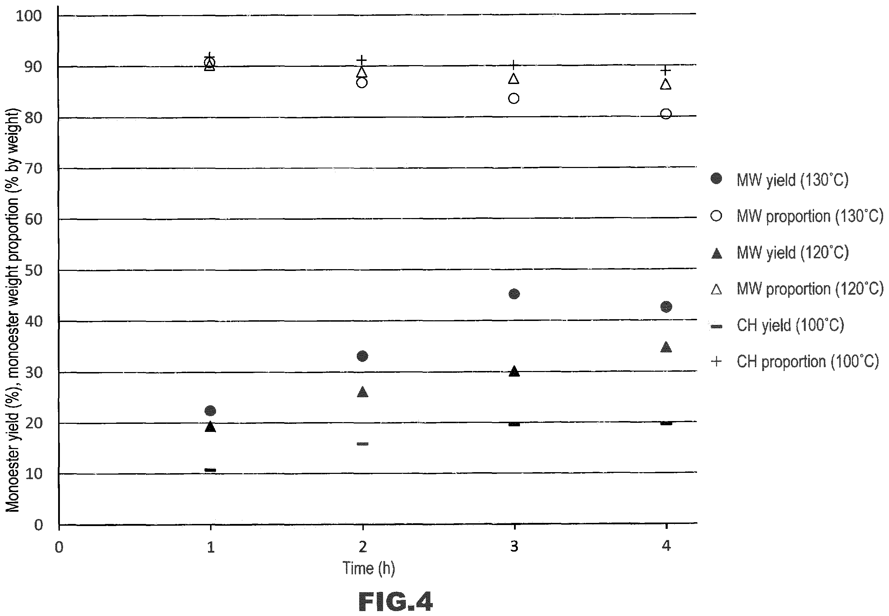

FIG. 4 is a graph showing time-series changes in the monoester yields and the monoester weight proportions of Examples 8 to 15 and Comparative Examples 6 to 9. In production of a sucrose stearic acid ester, the reaction progresses such that a monoester is first produced, a diester is produced from the monoester, and a triester is produced from the diester, and thus, as shown in the changes in the yields in Examples 8 to 11, a monoester yield gradually increases in accordance with the elapse of time, and takes a downward turn at a certain point during the reaction. Furthermore, since a diester and the like are produced in accordance with the elapse of time, a monoester proportion gradually decreases from 100%.

In FIG. 4, for example, a comparison between the microwave heating at 130°C (Examples 8 to 11) and the conventional heating at 100°C (Comparative Examples 6 to 9) shows that the time of the time-series change in the monoester yield in the microwave heating was shortened to be smaller than 1/4 the time of the time-series change in the monoester yield in the conventional heating. The reason for this is that, assuming that the change in the yield up to 4 hours in the conventional heating was a monotonic increase, the microwave heating time corresponding to the yield at 4 hours in the conventional heating was shorter than 1 hour. On the other hand, it is seen that the time of the time-series change in the monoester proportion in the microwave heating was not shortened to be 1/4 the time of the time-series change in the monoester proportion in the conventional heating. The reason for this is that the monoester weight proportion (88.9%) in Comparative Example 9 was smaller than the monoester weight proportion (90.7%) in Example 8. In this manner, it is seen that, in the reaction that sequentially esterifies multiple hydroxyl groups of sucrose, if the conventional heating is changed to the microwave heating, the monoester yield is shortened to be shorter in the time direction than the monoester proportion. Due to such a difference, the microwave heating can simultaneously realize both a high monoester yield and a high monoester selectivity in the range of a short reaction time (e.g., the range of a reaction time of 4 hours or shorter, etc.).

Referring to FIG. 4, it is seen that the monoester yield in the microwave heating at 130°C (Examples 8 to 11) had a peak with a heating time of approximately 3 hours. Accordingly, the transesterification time (i.e., the heating time of the subsequent step) may be set to a time at which the monoester yield reaches a peak. Note that the number of points in time at which the monoester yield reaches a peak is to be theoretically one, but it is actually difficult to specify the point in time. Accordingly, the time at which the monoester yield reaches a peak may be considered as a period of time having a width. For example, the transesterification time at which the monoester yield reached a peak in the microwave heating at 130°C (Examples 8 to 11) may be considered to be in the range of 2.5 hours to 3.5 hours.

Furthermore, a comparison between Examples 8 to 11 and Examples 12 to 15 shows that, in the range of a temperature that is below the sucrose decomposition temperature, a higher monoester yield can be realized at a higher reaction temperature. Accordingly, it is seen that the reaction is caused to occur preferably at a reaction temperature that is closer to the sucrose decomposition temperature.

### 3. Method for Producing Sucrose Caprylic Acid Ester

### Example 16

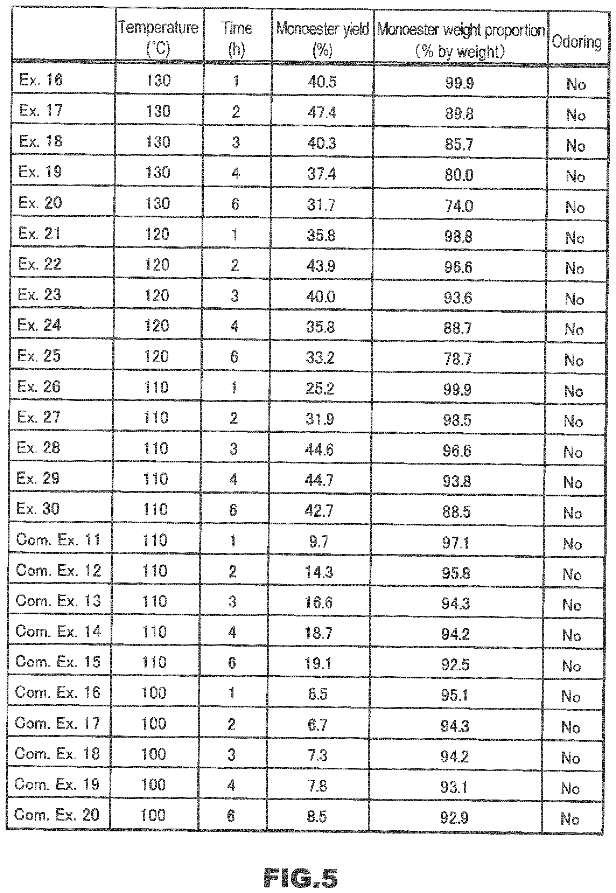

In this example, 57.5 g of methyl caprylate melted by being heated to 60°C and a solution in which 3.8 g of potassium hydroxide was dissolved in 30 ml of methanol were added to a three-necked flask. After the three-necked flask was placed in a microwave reactor (µReactor Ex, manufactured by Shikoku Instrumentation Co., Ltd.) provided with a stirrer and a thermometer, the content was irradiated with microwaves at 2.45 GHz, heated to 100°C with stirring, and refluxed for 10 minutes. Then, the temperature was kept at 100°C while reducing the pressure to 10 kPa, so that potassium caprylate was produced. After methanol was sufficiently removed by maintaining this state, the content was cooled down to 80°C. The content was a mixture of 46.8 g of methyl caprylate and 12.3 g of potassium caprylate. An aqueous solution in which 12 g of sucrose and 0.5 g of potassium hydroxide were dissolved in 8 g of water was added to the three-necked flask. While keeping the mixture in the three-necked flask at 80°C, the pressure was reduced to 10 kPa with stirring, so that water was evaporated. Then, while maintaining the stirring and the reduced pressure, microwaves were irradiated to increase the temperature to 130°C, and transesterification was performed for 1 hour, so that a sucrose caprylic acid ester was produced. Note that there was no odoring from the start to the end of the reaction. That is to say, during the reaction, the temperature was kept below a temperature at which sucrose starts to decompose. After the reaction ended, acetone was used to obtain a sucrose caprylic acid ester from the reacted material. The yield and the monoester proportion of the sucrose caprylic acid ester were analyzed using high performance liquid chromatography and gas chromatography. The results are as shown in FIG. 5.

### Examples 17 to 20

Sucrose caprylic acid esters were produced as in Example 16, except that the transesterification time was changed from 1 hour to 2 hours, 3 hours, 4 hours, or 6 hours. Also in this case, there was no odoring from the start to the end of the reaction. The results are as shown in FIG. 5.

### Examples 21 to 25

Sucrose caprylic acid esters were produced as in Examples 16 to 20, except that the temperature in the transesterification was changed from 130°C to 120°C. Also in this case, there was no odoring from the start to the end of the reaction. The results are as shown in FIG. 5.

### Examples 26 to 30

Sucrose caprylic acid esters were produced as in Examples 16 to 20, except that the temperature in the transesterification was changed from 130°C to 110°C. Also in this case, there was no odoring from the start to the end of the reaction. The results are as shown in FIG. 5.

### Comparative Examples 11 to 15

Sucrose caprylic acid esters were produced as in Examples 26 to 30, except that the heating with microwave irradiation was changed to heating with an oil bath (conventional heating). In this case, there was no odoring from the start to the end of the reaction. The results are as shown in FIG. 5.

When the temperature in the transesterification was set to 130°C in the heating with an oil bath, the reacted liquid was burnt black, and there was a smell of burnt sucrose. Furthermore, when the temperature in the transesterification was set to 120°C in the heating with an oil bath, the reacted liquid was burnt black after heating for 2 hours, and there was a smell of burnt sucrose. Accordingly, if heating is performed at a temperature of 120°C or more in the conventional heating, it is difficult to produce a sucrose caprylic acid ester with no odoring.

### Comparative Examples 16 to 20

Sucrose caprylic acid esters were produced as in Comparative Examples 11 to 15, except that the temperature in the transesterification was changed from 110°C to 100°C. Also in this case, there was no odoring from the start to the end of the reaction. The results are as shown in FIG. 5.

In order to prevent odoring in conventional heating, the temperature has to be lowered. Furthermore, if a sucrose caprylic acid is produced at a lower temperature in the conventional heating, as can be seen from the results of Comparative Examples 11 to 20, the monoester weight proportion is lower if the monoester yield is higher, and the monoester yield is lower if the monoester weight proportion is higher. On the other hand, in the case of Examples 16 to 30 where heating was performed with microwaves, both a high monoester yield and a high monoester weight proportion were realized compared with Comparative Examples 11 to 20. For example, yields higher than those in the conventional heating were realized with the microwave heating in all of Examples 16 to 30, whereas, in the conventional heating, time-series changes in the monoester yield were gradual, and thus a high monoester yield was not realized. Furthermore, with the microwave heating, such a high yield was realized without causing odoring. Furthermore, in Examples 17 and 22, high monoester yields and high monoester weight proportions were realized in a short time.

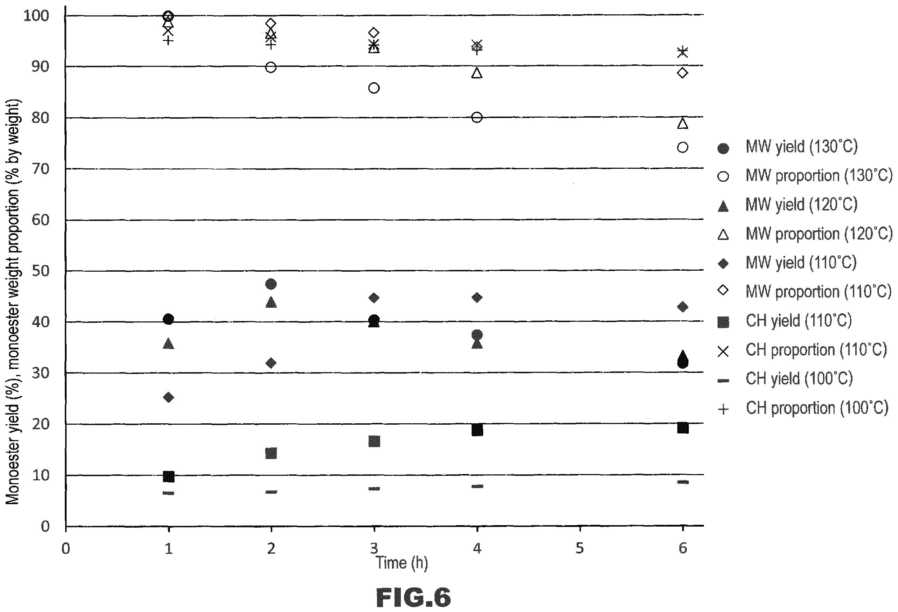

FIG. 6 is a graph showing time-series changes in the monoester yields and the monoester weight proportions of Examples 16 to 30 and Comparative Examples 11 to 20. In production of a sucrose caprylic acid ester, the reaction progresses such that a monoester is first produced, a diester is produced from the monoester, and a triester is produced from the diester, and thus, as shown in the changes in the yields in Examples 16 to 30, a monoester yield gradually increases in accordance with the elapse of time, and takes a downward turn at a certain point during the reaction. Furthermore, since a diester and the like are produced in accordance with the elapse of time, a monoester proportion gradually decreases from 100%.

In FIG. 6, for example, a comparison between the microwave heating at 130°C (Examples 16 to 20) and the conventional heating at 110°C (Comparative Examples 11 to 15) shows that the time of the time-series change in the monoester yield in the microwave heating was shortened to be smaller than 1/8 the time of the time-series change in the monoester yield in the conventional heating. The reason for this is that, assuming that both the change in the yield up to 1 hour in the microwave heating and the change in the yield up to 4 hours in the conventional heating were monotonic increases, the microwave heating time corresponding to the yield at 4 hours in the conventional heating was shorter than 0.5 hours. On the other hand, it is seen that the time of the time-series change in the monoester proportion in the microwave heating was not shortened to be 1/8 the time of the time-series change in the monoester proportion in the conventional heating. The reason for this is that the monoester weight proportion (92.5%) in Comparative Example 15 was smaller than the monoester weight proportion (99.9%) in Example 16. In this manner, it is seen that, in the reaction that sequentially esterifies multiple hydroxyl groups of sucrose, if the conventional heating is changed to the microwave heating, the monoester yield is shortened to be shorter in the time direction than the monoester proportion. Due to such a difference, the microwave heating can simultaneously realize both a high monoester yield and a high monoester selectivity in the range of a short reaction time (e.g., the range of a reaction time of 6 hours or shorter, etc.).

Referring to FIG. 6, it is seen that the monoester yield in the microwave heating at 130°C (Examples 16 to 20) had a peak with a heating time of approximately 2 hours. Accordingly, the transesterification time (i.e., the heating time of the subsequent step) may be set to a time at which the monoester yield reaches a peak. Note that the number of points in time at which the monoester yield reaches a peak is to be theoretically one, but it is actually difficult to specify the point in time. Accordingly, the time at which the monoester yield reaches a peak may be considered as a period of time having a width. For example, the transesterification time at which the monoester yield reached a peak in the microwave heating at 130°C may be considered to be in the range of 1.5 hours to 2.5 hours.

Furthermore, a comparison between Examples 16 to 20 and Examples 21 to 25 shows that, in the range of a temperature that is below the sucrose decomposition temperature, a higher monoester yield can be realized at a higher reaction temperature. Accordingly, it seems that the reaction is caused to occur preferably at a reaction temperature that is closer to the sucrose decomposition temperature. Furthermore, in this example, if the heating time in the subsequent step is 6 hours or less, it is possible to realize a monoester weight proportion of 70% by weight or more. In particular, if the heating time in the subsequent step is 4 hours or less, it is possible to realize a monoester weight proportion of 80% by weight or more.

### 4. Method for Producing Sucrose Lauric Acid Ester

### Example 31

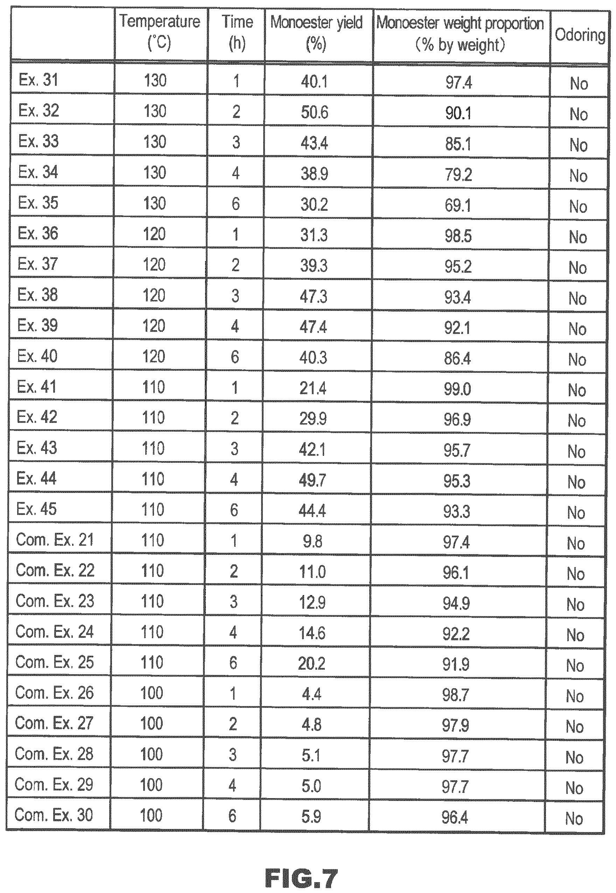

In this example, 77.9 g of methyl laurate melted by being heated to 60°C and a solution in which 3.8 g of potassium hydroxide was dissolved in 30 ml of methanol were added to a three-necked flask. After the three-necked flask was placed in a microwave reactor (µReactor Ex, manufactured by Shikoku Instrumentation Co., Ltd.) provided with a stirrer and a thermometer, the content was irradiated with microwaves at 2.45 GHz, heated to 100°C with stirring, and refluxed for 10 minutes. Then, the temperature was kept at 100°C while reducing the pressure to 4 kPa, so that potassium laurate was produced. After methanol was sufficiently removed by maintaining this state, the content was cooled down to 80°C. The content was a mixture of 63.4 g of methyl laurate and 16.1 g of potassium laurate. An aqueous solution in which 12 g of sucrose and 0.5 g of potassium hydroxide were dissolved in 8 g of water was added to the three-necked flask. While keeping the mixture in the three-necked flask at 80°C, the pressure was reduced to 4 kPa with stirring, so that water was evaporated. Then, while maintaining the stirring and the reduced pressure, microwaves were irradiated to increase the temperature to 130°C, and transesterification was performed for 1 hour, so that a sucrose lauric acid ester was produced. Note that there was no odoring from the start to the end of the reaction. That is to say, during the reaction, the temperature was kept below a temperature at which sucrose starts to decompose. After the reaction ended, acetone was used to obtain a sucrose lauric acid ester from the reacted material. The yield and the monoester proportion of the sucrose lauric acid ester were analyzed using high performance liquid chromatography and gas chromatography. The results are as shown in FIG. 7.

### Examples 32 to 35

Sucrose lauric acid esters were produced as in Example 31, except that the transesterification time was changed from 1 hour to 2 hours, 3 hours, 4 hours, or 6 hours. Also in this case, there was no odoring from the start to the end of the reaction. The results are as shown in FIG. 7.

### Examples 36 to 40

Sucrose lauric acid esters were produced as in Examples 31 to 35, except that the temperature in the transesterification was changed from 130°C to 120°C. Also in this case, there was no odoring from the start to the end of the reaction. The results are as shown in FIG. 7.

### Examples 41 to 45

Sucrose lauric acid esters were produced as in Examples 31 to 35, except that the temperature in the transesterification was changed from 130°C to 110°C. Also in this case, there was no odoring from the start to the end of the reaction. The results are as shown in FIG. 7.

### Comparative Examples 21 to 25

Sucrose lauric acid esters were produced as in Examples 41 to 45, except that the heating with microwave irradiation was changed to heating with an oil bath (conventional heating). In this case, there was no odoring from the start to the end of the reaction. The results are as shown in FIG. 7.

When the temperature in the transesterification was set to 130°C in the heating with an oil bath, the reacted liquid was burnt black, and there was a smell of burnt sucrose. Furthermore, when the temperature in the transesterification was set to 120°C in the heating with an oil bath, there was a smell of burnt sucrose in the reacted liquid after heating for 1 hour. Accordingly, if heating is performed at a temperature of 120°C or more in the conventional heating, it is difficult to produce a sucrose lauric acid ester with no odoring.

### Comparative Examples 26 to 30

Sucrose lauric acid esters were produced as in Comparative Examples 21 to 25, except that the temperature in the transesterification was changed from 110°C to 100°C. Also in this case, there was no odoring from the start to the end of the reaction. The results are as shown in FIG. 7.

In order to prevent odoring in conventional heating, the temperature has to be lowered. Furthermore, if a sucrose lauric acid is produced at a lower temperature in the conventional heating, as can be seen from the results of Comparative Examples 21 to 30, the monoester weight proportion is lower if the monoester yield is higher, and the monoester yield is lower if the monoester weight proportion is higher. On the other hand, in the case of Examples 31 to 45 where heating was performed with microwaves, both a high monoester yield and a high monoester weight proportion were realized compared with Comparative Examples 21 to 30. For example, yields higher than those in the conventional heating were realized with the microwave heating in all of Examples 31 to 45, whereas, in the conventional heating, time-series changes in the monoester yield were gradual, and thus a high monoester yield was not realized. Furthermore, with the microwave heating, such a high yield was realized without causing odoring. Furthermore, in Examples 32 and 38, high monoester yields and high monoester weight proportions were realized in a short time.

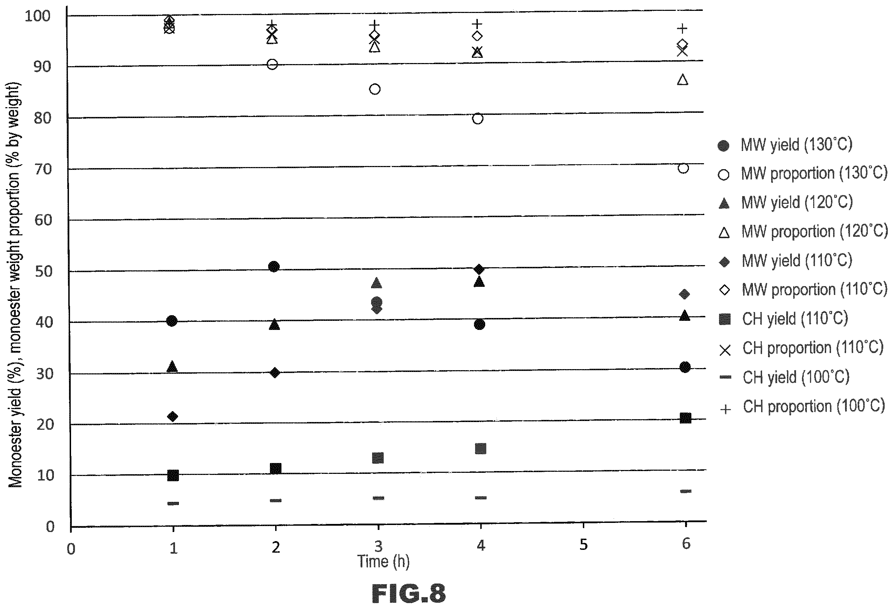

FIG. 8 is a graph showing time-series changes in the monoester yields and the monoester weight proportions of Examples 31 to 45 and Comparative Examples 21 to 30. In production of a sucrose lauric acid ester, the reaction progresses such that a monoester is first produced, a diester is produced from the monoester, and a triester is produced from the diester, and thus, as shown in the changes in the yields in Examples 31 to 45, a monoester yield gradually increases in accordance with the elapse of time, and takes a downward turn at a certain point during the reaction. Furthermore, since a diester and the like are produced in accordance with the elapse of time, a monoester proportion gradually decreases from 100%.

In FIG. 8, for example, a comparison between the microwave heating at 130°C (Examples 31 to 35) and the conventional heating at 110°C (Comparative Examples 21 to 25) shows that the time of the time-series change in the monoester yield in the microwave heating was shortened to be approximately 1/12 the time of the time-series change in the monoester yield in the conventional heating. The reason for this is that, assuming that both the change in the yield up to 1 hour in the microwave heating and the change in the yield up to 6 hours in the conventional heating were monotonic increases, the microwave heating time corresponding to the yield at 6 hours in the conventional heating is approximately 0.5 hours. On the other hand, it is seen that the time of the time-series change in the monoester proportion in the microwave heating was not shortened to be 1/12 the time of the time-series change in the monoester proportion in the conventional heating. The reason for this is that the monoester weight proportion (91.9%) in Comparative Example 25 was smaller than the monoester weight proportion (97.4%) in Example 31. In this manner, it is seen that, in the reaction that sequentially esterifies multiple hydroxyl groups of sucrose, if the conventional heating is changed to the microwave heating, the monoester yield is shortened to be shorter in the time direction than the monoester proportion. Due to such a difference, the microwave heating can simultaneously realize both a high monoester yield and a high monoester selectivity in the range of a short reaction time (e.g., the range of a reaction time of 6 hours or shorter, etc.).

Referring to FIG. 8, it is seen that the monoester yield in the microwave heating at 130°C (Examples 31 to 35) had a peak with a heating time of approximately 2 hours. Accordingly, the transesterification time (i.e., the heating time of the subsequent step) may be set to a time at which the monoester yield reaches a peak. Note that the number of points in time at which the monoester yield reaches a peak is to be theoretically one, but it is actually difficult to specify the point in time. Accordingly, the time at which the monoester yield reaches a peak may be considered as a period of time having a width. For example, the transesterification time at which the monoester yield reached a peak in the microwave heating at 130°C may be considered to be in the range of 1.5 hours to 2.5 hours.

Furthermore, a comparison between Examples 31 to 35 and Examples 36 to 40 shows that, in the range of a temperature that is below the sucrose decomposition temperature, a higher monoester yield can be realized at a higher reaction temperature. Accordingly, it seems that the reaction is caused to occur preferably at a reaction temperature that is closer to the sucrose decomposition temperature. Furthermore, in this example, if the heating time in the subsequent step is 6 hours or less, it is possible to realize a monoester weight proportion of 65% by weight or more. In particular, if the heating time in the subsequent step is 4 hours or less, it is possible to realize a monoester weight proportion of 75% by weight or more.

### 5. Method for Producing Sucrose Oleic Acid Ester

### Example 46

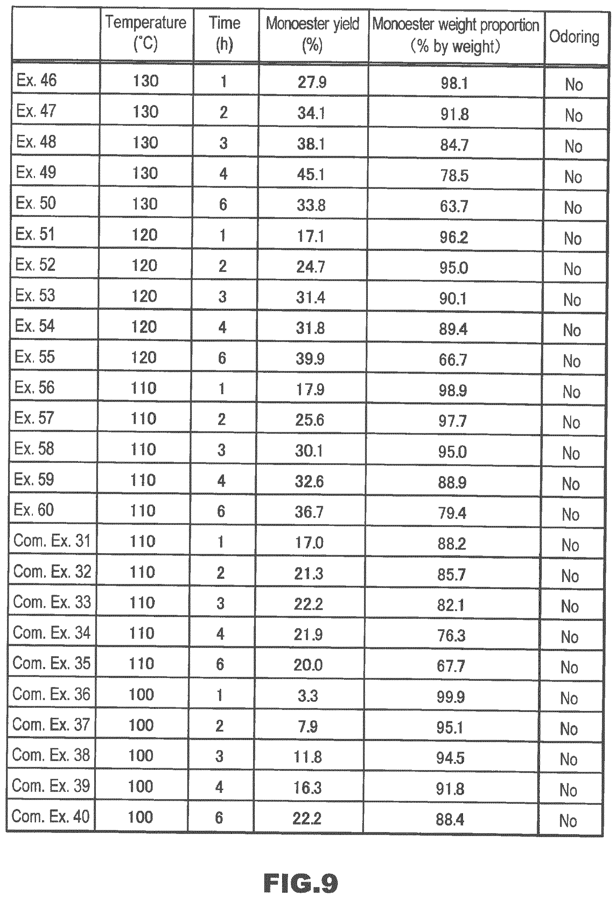

In this example, 107.8 g of methyl oleate melted by being heated to 60°C and a solution in which 3.8 g of potassium hydroxide was dissolved in 30 ml of methanol were added to a three-necked flask. After the three-necked flask was placed in a microwave reactor (µReactor Ex, manufactured by Shikoku Instrumentation Co., Ltd.) provided with a stirrer and a thermometer, the content was irradiated with microwaves at 2.45 GHz, heated to 100°C with stirring, and refluxed for 10 minutes. Then, the temperature was kept at 100°C while reducing the pressure to 4 kPa, so that potassium oleate was produced. After methanol was sufficiently removed by maintaining this state, the content was cooled down to 80°C. The content was a mixture of 87.7 g of methyl oleate and 21.7 g of potassium oleate. An aqueous solution in which 12 g of sucrose and 0.5 g of potassium hydroxide were dissolved in 8 g of water was added to the three-necked flask. While keeping the mixture in the three-necked flask at 80°C, the pressure was reduced to 4 kPa with stirring, so that water was evaporated. Then, while maintaining the stirring and the reduced pressure, microwaves were irradiated to increase the temperature to 130°C, and transesterification was performed for 1 hour, so that a sucrose oleic acid ester was produced. Note that there was no odoring from the start to the end of the reaction. That is to say, during the reaction, the temperature was kept below a temperature at which sucrose starts to decompose. After the reaction ended, acetone was used to obtain a sucrose oleic acid ester from the reacted material. The yield and the monoester proportion of the sucrose oleic acid ester were analyzed using high performance liquid chromatography and gas chromatography. The results are as shown in FIG. 9.

### Examples 47 to 50

Sucrose oleic acid esters were produced as in Example 46, except that the transesterification time was changed from 1 hour to 2 hours, 3 hours, 4 hours, or 6 hours. Also in this case, there was no odoring from the start to the end of the reaction. The results are as shown in FIG. 9.

### Examples 51 to 55

Sucrose oleic acid esters were produced as in Examples 46 to 50, except that the temperature in the transesterification was changed from 130°C to 120°C. Also in this case, there was no odoring from the start to the end of the reaction. The results are as shown in FIG. 9.

### Examples 56 to 60

Sucrose oleic acid esters were produced as in Examples 46 to 50, except that the temperature in the transesterification was changed from 130°C to 110°C. Also in this case, there was no odoring from the start to the end of the reaction. The results are as shown in FIG. 9.

### Comparative Examples 31 to 35

Sucrose oleic acid esters were produced as in Examples 56 to 60, except that the heating with microwave irradiation was changed to heating with an oil bath (conventional heating). In this case, there was no odoring from the start to the end of the reaction. The results are as shown in FIG. 9.

When the temperature in the transesterification was set to 130°C in the heating with an oil bath, the reacted liquid was burnt black, and there was a smell of burnt sucrose. Furthermore, when the temperature in the transesterification was set to 120°C in the heating with an oil bath, there was a smell of burnt sucrose in the reacted liquid after heating for 1.5 hours. Accordingly, if heating is performed at a temperature of 120°C or more in the conventional heating, it is difficult to produce a sucrose oleic acid ester with no odoring.

### Comparative Examples 36 to 40

Sucrose oleic acid esters were produced as in Comparative Examples 31 to 35, except that the temperature in the transesterification was changed from 110°C to 100°C. Also in this case, there was no odoring from the start to the end of the reaction. The results are as shown in FIG. 9.

In order to prevent odoring in conventional heating, the temperature has to be lowered. Furthermore, if a sucrose oleic acid is produced at a lower temperature in the conventional heating, as can be seen from the results of Comparative Examples 31 to 40, the monoester weight proportion is lower if the monoester yield is higher, and the monoester yield is lower if the monoester weight proportion is higher. On the other hand, in the case of Examples 46 to 60 where heating was performed with microwaves, both a high monoester yield and a high monoester weight proportion were realized compared with Comparative Examples 31 to 40. For example, a comparison between the monoester yields in Example 47 and Comparative Example 39 having the same monoester weight proportion shows that, with the microwave heating, a yield that was 2.1 times as high as that of the conventional heating was realized. Furthermore, with the microwave heating, such a high yield was realized without causing odoring. In the conventional heating, time-series changes in the monoester yield were gradual or had low upper limits, and thus a high monoester yield was not realized, whereas, in Examples 46 to 50, etc., yields higher than those in the comparative examples were realized. In particular in Examples 48 and 49, high monoester yields and high monoester weight proportions were realized in a short time.

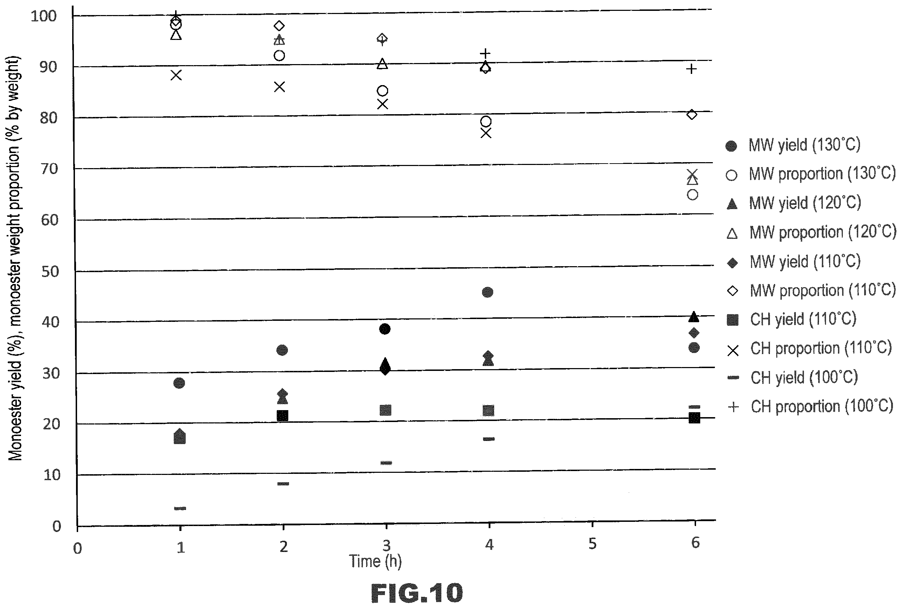

FIG. 10 is a graph showing time-series changes in the monoester yields and the monoester weight proportions of Examples 46 to 60 and Comparative Examples 31 to 40. In production of a sucrose oleic acid ester, the reaction progresses such that a monoester is first produced, a diester is produced from the monoester, and a triester is produced from the diester, and thus, as shown in the changes in the yields in Examples 46 to 50, a monoester yield gradually increases in accordance with the elapse of time, and takes a downward turn at a certain point during the reaction. Furthermore, since a diester and the like are produced in accordance with the elapse of time, a monoester proportion gradually decreases from 100%.

In FIG. 10, the time-series change in the conventional heating at 110°C (Comparative Examples 31 to 35) had an inclination different from that of the time-series change in the conventional heating in Comparative Examples 1 to 30, and, in the heating initial stage, the degree of increase in the monoester yield was large, and the degree of decrease in the monoester proportion was also large. However, in the conventional heating in Comparative Examples 31 to 35, the monoester yield reached the upper limit in approximately 3 hours, and the upper limit value was lower than the upper limit value of the monoester yield in the microwave heating. For example, a comparison between Example 49 and Comparative Example 33 shows that the monoester yield in the conventional heating was approximately half the monoester yield in the microwave heating. Furthermore, the time-series change in the monoester proportion in the microwave heating had values as a whole larger than those of the time-series change in the monoester proportion in the conventional heating in Comparative Examples 31 to 35. Accordingly, in the case of the microwave heating, a higher monoester yield and a higher monoester selectivity were realized compared with the conventional heating at 110°C.

In FIG. 10, for example, a comparison between the microwave heating at 130°C (Examples 46 to 50) and the conventional heating at 100°C (Comparative Examples 36 to 40) shows that the time of the time-series change in the monoester yield in the microwave heating was shortened to be smaller than 1/6 the time of the time-series change in the monoester yield in the conventional heating. The reason for this is that, assuming that the change in the yield up to 1 hour in the microwave heating and the change in the yield up to 6 hours in the conventional heating were monotonic increases, the microwave heating time corresponding to the yield at 6 hours in the conventional heating was shorter than 1 hour. On the other hand, it is seen that the time of the time-series change in the monoester proportion in the microwave heating was not shortened to be 1/6 the time of the time-series change in the monoester proportion in the conventional heating. The reason for this is that the monoester weight proportion (88.4%) in Comparative Example 40 was smaller than the monoester weight proportion (91.8%) in Example 47. In this manner, it is seen that, in the reaction that sequentially esterifies multiple hydroxyl groups of sucrose, if the conventional heating is changed to the microwave heating, the monoester yield is shortened to be shorter in the time direction than the monoester proportion. Due to such a difference, the microwave heating can simultaneously realize both a high monoester yield and a high monoester selectivity in the range of a short reaction time (e.g., the range of a reaction time of 6 hours or shorter, in particular 4 hours or shorter, etc.).

Referring to FIG. 10, it is seen that the monoester yield in the microwave heating at 130°C (Examples 46 to 50) had a peak with a heating time of approximately 4 hours. Accordingly, the transesterification time (i.e., the heating time of the subsequent step) may be set to a time at which the monoester yield reaches a peak. Note that the number of points in time at which the monoester yield reaches a peak is to be theoretically one, but it is actually difficult to specify the point in time. Accordingly, the time at which the monoester yield reaches a peak may be considered as a period of time having a width. For example, the transesterification time at which the monoester yield reached a peak in the microwave heating at 130°C (Examples 46 to 50) may be considered to be in the range of 3.5 hours to 5 hours.

Furthermore, a comparison between Examples 46 to 50 and Examples 51 to 55 shows that, in the range of a temperature that is below the sucrose decomposition temperature, a higher monoester yield can be realized at a higher reaction temperature. Accordingly, it seems that the reaction is caused to occur preferably at a reaction temperature that is closer to the sucrose decomposition temperature. Furthermore, in this example, if the heating time in the subsequent step is 6 hours or less, it is possible to realize a monoester weight proportion of 60% by weight or more. In particular, if the heating time in the subsequent step is 4 hours or less, it is possible to realize a monoester weight proportion of 70% by weight or more.

### 6. Method for Producing Sucrose Linoleic Acid Ester

### Example 61

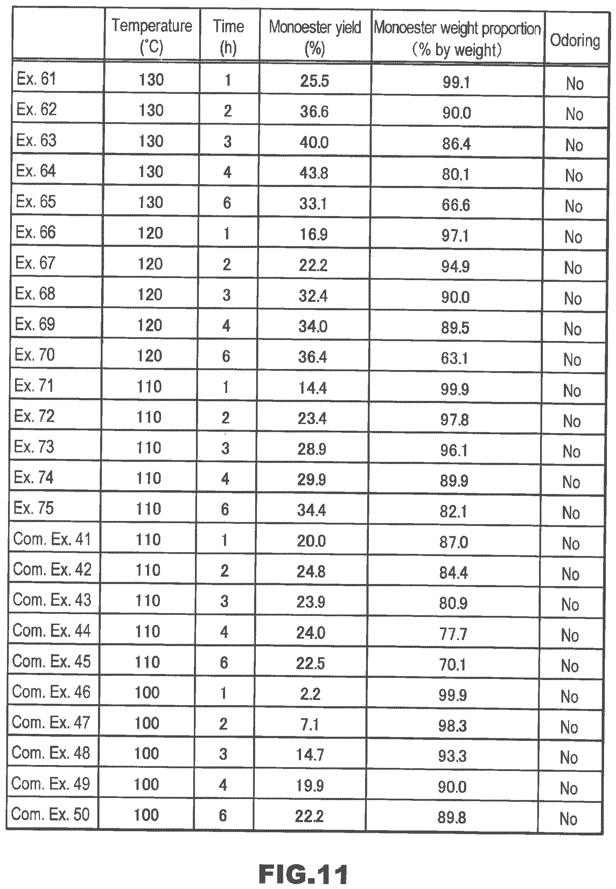

In this example, 107.1 g of methyl linoleate melted by being heated to 60°C and a solution in which 3.8 g of potassium hydroxide was dissolved in 30 ml of methanol were added to a three-necked flask. After the three-necked flask was placed in a microwave reactor (µReactor Ex, manufactured by Shikoku Instrumentation Co., Ltd.) provided with a stirrer and a thermometer, the content was irradiated with microwaves at 2.45 GHz, heated to 100°C with stirring, and refluxed for 10 minutes. Then, the temperature was kept at 100°C while reducing the pressure to 4 kPa, so that potassium linoleate was produced. After methanol was sufficiently removed by maintaining this state, the content was cooled down to 80°C. The content was a mixture of 87.1 g of methyl linoleate and 21.6 g of potassium linoleate. An aqueous solution in which 12 g of sucrose and 0.5 g of potassium hydroxide were dissolved in 8 g of water was added to the three-necked flask. While keeping the mixture in the three-necked flask at 80°C, the pressure was reduced to 4 kPa with stirring, so that water was evaporated. Then, while maintaining the stirring and the reduced pressure, microwaves were irradiated to increase the temperature to 130°C, and transesterification was performed for 1 hour, so that a sucrose linoleic acid ester was produced. Note that there was no odoring from the start to the end of the reaction. That is to say, during the reaction, the temperature was kept below a temperature at which sucrose starts to decompose. After the reaction ended, acetone was used to obtain a sucrose linoleic acid ester from the reacted material. The yield and the monoester proportion of the sucrose linoleic acid ester were analyzed using high performance liquid chromatography and gas chromatography. The results are as shown in FIG. 11.

### Examples 62 to 65

Sucrose linoleic acid esters were produced as in Example 61, except that the transesterification time was changed from 1 hour to 2 hours, 3 hours, 4 hours, or 6 hours. Also in this case, there was no odoring from the start to the end of the reaction. The results are as shown in FIG. 11.

### Examples 66 to 70

Sucrose linoleic acid esters were produced as in Examples 61 to 65, except that the temperature in the transesterification was changed from 130°C to 120°C. Also in this case, there was no odoring from the start to the end of the reaction. The results are as shown in FIG. 11.

### Examples 71 to 75

Sucrose linoleic acid esters were produced as in Examples 61 to 65, except that the temperature in the transesterification was changed from 130°C to 110°C. Also in this case, there was no odoring from the start to the end of the reaction. The results are as shown in FIG. 11.

### Comparative Examples 41 to 45

Sucrose linoleic acid esters were produced as in Examples 71 to 75, except that the heating with microwave irradiation was changed to heating with an oil bath (conventional heating). In this case, there was no odoring from the start to the end of the reaction. The results are as shown in FIG. 11.

When the temperature in the transesterification was set to 130°C in the heating with an oil bath, the reacted liquid was burnt black, and there was a smell of burnt sucrose. Furthermore, when the temperature in the transesterification was set to 120°C in the heating with an oil bath, there was a smell of burnt sucrose in the reacted liquid after heating for 1.5 hours. Accordingly, if heating is performed at a temperature of 120°C or more in the conventional heating, it is difficult to produce a sucrose linoleic acid ester with no odoring.

### Comparative Examples 46 to 50

Sucrose linoleic acid esters were produced as in Comparative Examples 41 to 45, except that the temperature in the transesterification was changed from 110°C to 100°C. Also in this case, there was no odoring from the start to the end of the reaction. The results are as shown in FIG. 11.

In order to prevent odoring in conventional heating, the temperature has to be lowered. Furthermore, if a sucrose linoleic acid is produced at a lower temperature in the conventional heating, as can be seen from the results of Comparative Examples 41 to 50, the monoester weight proportion is lower if the monoester yield is higher, and the monoester yield is lower if the monoester weight proportion is higher. On the other hand, in the case of Examples 61 to 75 where heating was performed with microwaves, both a high monoester yield and a high monoester weight proportion were realized compared with Comparative Examples 41 to 50. For example, a comparison between the monoester yields in Example 62 and Comparative Example 49 having the same monoester weight proportion shows that, with the microwave heating, a yield that was 1.8 times as high as that of the conventional heating was realized. Furthermore, with the microwave heating, such a high yield was realized without causing odoring. In the conventional heating, time-series changes in the monoester yield were gradual or had low upper limits, and thus a high monoester yield was not realized, whereas, in Examples 61 to 65, etc., yields higher than those in the comparative examples were realized. In particular in Examples 63 and 64, high monoester yields and high monoester weight proportions were realized in a short time.

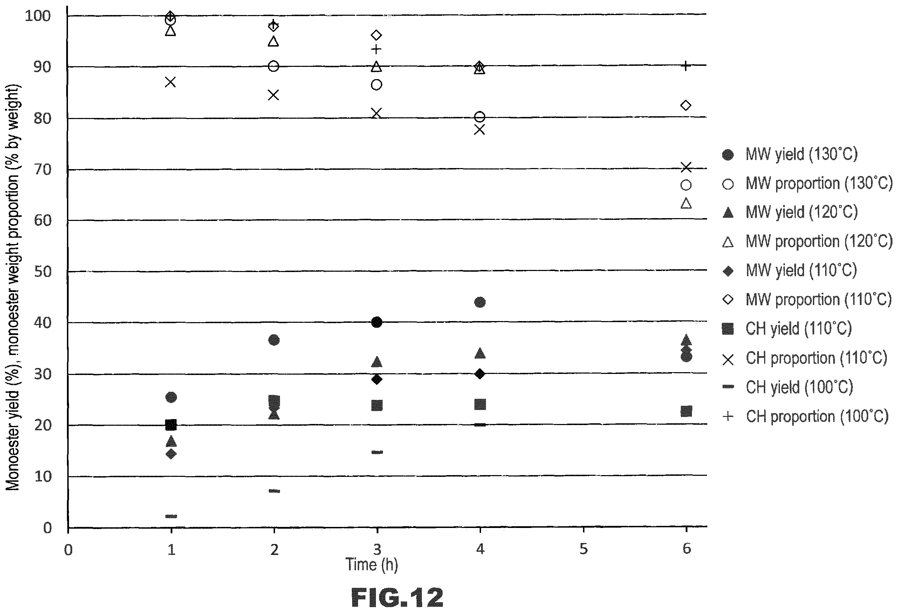

FIG. 12 is a graph showing time-series changes in the monoester yields and the monoester weight proportions of Examples 61 to 75 and Comparative Examples 41 to 50. In production of a sucrose linoleic acid ester, the reaction progresses such that a monoester is first produced, a diester is produced from the monoester, and a triester is produced from the diester, and thus, as shown in the changes in the yields in Examples 61 to 65, a monoester yield gradually increases in accordance with the elapse of time, and takes a downward turn at a certain point during the reaction. Furthermore, since a diester and the like are produced in accordance with the elapse of time, a monoester proportion gradually decreases from 100%.

In FIG. 12, the time-series change the conventional heating at 110°C (Comparative Examples 41 to 45) had an inclination different from that of the time-series change in the conventional heating in Comparative Examples 1 to 30, and, in the heating initial stage, the degree of increase in the monoester yield was large, and the degree of decrease in the monoester proportion was also large. However, in the conventional heating in Comparative Examples 41 to 45, the monoester yield reached the upper limit in approximately 2 hours, and the upper limit value was lower than the upper limit value of the monoester yield in the microwave heating. For example, a comparison between Example 64 and Comparative Example 42 shows that the monoester yield in the conventional heating was approximately half (about 0.57) the monoester yield in the microwave heating. Furthermore, the time-series change in the monoester proportion in the microwave heating had values as a whole larger than those of the time-series change in the monoester proportion in the conventional heating in Comparative Examples 41 to 45. Accordingly, in the case of the microwave heating, a higher monoester yield and a higher monoester selectivity were realized compared with the conventional heating at 110°C.

In FIG. 12, for example, a comparison between the microwave heating at 130°C (Examples 61 to 65) and the conventional heating at 100°C (Comparative Examples 46 to 50) shows that the time of the time-series change in the monoester yield in the microwave heating was shortened to be smaller than 1/6 the time of the time-series change in the monoester yield in the conventional heating. The reason for this is that, assuming that the change in the yield up to 1 hour in the microwave heating and the change in the yield up to 6 hours in the conventional heating were monotonic increases, the microwave heating time corresponding to the yield at 6 hours in the conventional heating was shorter than 1 hour. On the other hand, it is seen that the time of the time-series change in the monoester proportion in the microwave heating was not shortened to be 1/6 the time of the time-series change in the monoester proportion in the conventional heating. The reason for this is that the monoester weight proportion (89.8%) in Comparative Example 50 was smaller than the monoester weight proportion (90.0%) in Example 62. In this manner, it is seen that, in the reaction that sequentially esterifies multiple hydroxyl groups of sucrose, if the conventional heating is changed to the microwave heating, the monoester yield is shortened to be shorter in the time direction than the monoester proportion. Due to such a difference, the microwave heating can simultaneously realize both a high monoester yield and a high monoester selectivity in the range of a short reaction time (e.g., the range of a reaction time of 6 hours or shorter, in particular 4 hours or shorter, etc.).

Referring to FIG. 12, it is seen that the monoester yield in the microwave heating at 130°C (Examples 61 to 65) had a peak with a heating time of approximately 4 hours. Accordingly, the transesterification time (i.e., the heating time of the subsequent step) may be set to a time at which the monoester yield reaches a peak. Note that the number of points in time at which the monoester yield reaches a peak is to be theoretically one, but it is actually difficult to specify the point in time. Accordingly, the time at which the monoester yield reaches a peak may be considered as a period of time having a width. For example, the transesterification time at which the monoester yield reached a peak in the microwave heating at 130°C (Examples 61 to 65) may be considered to be in the range of 3.5 hours to 5 hours.

Furthermore, a comparison between Examples 61 to 65 and Examples 66 to 70 shows that, in the range of a temperature that is below the sucrose decomposition temperature, it can be inferred that a higher monoester yield can be realized at a higher reaction temperature. Accordingly, it seems that the reaction is caused to occur preferably at a reaction temperature that is closer to the sucrose decomposition temperature. Furthermore, in this example, if the heating time in the subsequent step is 6 hours or less, it is possible to realize a monoester weight proportion of 60% by weight or more. In particular, if the heating time in the subsequent step is 4 hours or less, it is possible to realize a monoester weight proportion of 70% by weight or more.

In the foregoing examples, it was described that, if the constituent fatty acid of the fatty acid ester is palmitic acid (with 16 carbon atoms; saturated fatty acid), stearic acid (with 18 carbon atoms; saturated fatty acid), caprylic acid (with 8 carbon atoms; saturated fatty acid), lauric acid (with 12 carbon atoms; saturated fatty acid), oleic acid (with 18 carbon atoms; mono-unsaturated fatty acid), or linoleic acid (with 18 carbon atoms; poly-unsaturated fatty acid), microwave irradiation makes it possible to produce, at a high yield, a sucrose fatty acid ester having a high monoester proportion and with no odoring. Accordingly, it can be inferred that similar effects can be obtained also in production of a sucrose fatty acid ester using a fatty acid ester whose constituent fatty acid is a fatty acid with 8 to 24 carbon atoms. Furthermore, it can be inferred that similar effects can be obtained also in the case where the constituent fatty acid of the fatty acid ester used in production of a sucrose fatty acid ester is either a saturated fatty acid other than those described above or an unsaturated fatty acid other than those described above. Moreover, it can be inferred that similar effects can be obtained regardless of the number of unsaturated bonds in the unsaturated fatty acid.

The present invention is not limited to the foregoing examples. Various modifications are possible within the scope of the present invention as defined in the appended claims.

### Industrial Applicability

The sucrose fatty acid ester obtained by the method for producing a sucrose fatty acid ester according to the present invention can be used in the fields of, for example, foods, cosmetics, drugs, and the like.

## Claims

1. A method for producing a sucrose fatty acid ester, comprising:
a step of preparing an aqueous solution containing sucrose and a basic catalyst; and
a step of producing a sucrose fatty acid ester, by mixing the aqueous solution obtained in the aforementioned step, a fatty acid alkali metal salt, and a fatty acid ester, and heating the mixture with stirring under reduced pressure,
wherein the step of producing a sucrose fatty acid ester has an earlier step of removing water from the mixture, and a subsequent step of performing transesterification after the earlier step, and
in the subsequent step, heating is performed through microwave irradiation such that the temperature of the mixture is below a decomposition temperature of the sucrose.

2. The method for producing a sucrose fatty acid ester according to claim 1, wherein a sucrose fatty acid ester with a monoester weight proportion of 60% by weight or more is produced.

3. The method for producing a sucrose fatty acid ester according to claim 1 or 2, wherein the fatty acid ester is a fatty acid ester whose constituent fatty acid includes at least one selected from the group consisting of saturated and unsaturated fatty acids with 8 to 24 carbon atoms.

4. The method for producing a sucrose fatty acid ester according to any one of claims 1 to 3, further comprising:
a step of producing a fatty acid alkali metal salt by mixing and heating a fatty acid ester and a solution in which an alkali metal salt is dissolved in solvent,
wherein the fatty acid alkali metal salt used in the step of producing a sucrose fatty acid ester has been produced in the step of producing a fatty acid alkali metal salt.

5. The method for producing a sucrose fatty acid ester according to any one of claims 1 to 4, wherein, in the subsequent step, the heating is performed such that the temperature of the mixture is at or above a temperature 25°C lower than the decomposition temperature, and is below the decomposition temperature.

6. The method for producing a sucrose fatty acid ester according to any one of claims 1 to 5, wherein, in the subsequent step, the heating is performed such that the temperature of the mixture increases.

7. The method for producing a sucrose fatty acid ester according to any one of claims 1 to 6, wherein the heating time of the subsequent step is 6 hours or less.

## Patentansprüche

1. Verfahren zur Herstellung eines Saccharosefettsäureesters, umfassend:
einen Schritt der Herstellung einer wässrigen Lösung, die Saccharose und einen basischen Katalysator enthält; und
einen Schritt zur Herstellung eines Saccharosefettsäureesters durch Mischen der in dem vorgenannten Schritt erhaltenen wässrigen Lösung, eines Fettsäurealkalimetallsalzes und eines Fettsäureesters und Erhitzen der Mischung unter Rühren unter vermindertem Druck,
wobei der Schritt der Herstellung eines Saccharosefettsäureesters einen früheren Schritt der Entfernung von Wasser aus der Mischung und einen nachfolgenden Schritt der Durchführung einer Umesterung nach dem früheren Schritt aufweist, und
im nachfolgenden Schritt erfolgt die Erwärmung durch Mikrowellenbestrahlung, so dass die Temperatur der Mischung unterhalb einer Zersetzungstemperatur der Saccharose liegt.

2. Verfahren zur Herstellung eines Saccharosefettsäureesters nach Anspruch 1, wobei ein Saccharosefettsäureester mit einem Monoester-Gewichtsanteil von 60 Gew.-% oder mehr hergestellt wird.

3. Verfahren zur Herstellung eines Saccharosefettsäureesters nach Anspruch 1 oder 2, wobei der Fettsäureester ein Fettsäureester ist, dessen Fettsäurebestandteil mindestens eine Fettsäure enthält, die aus der Gruppe ausgewählt ist, die aus gesättigten und ungesättigten Fettsäuren mit 8 bis 24 Kohlenstoffatomen besteht.

4. Verfahren zur Herstellung eines Saccharosefettsäureesters nach einem der Ansprüche 1 bis 3, das ferner umfasst:
einen Schritt der Herstellung eines Fettsäure-Alkalimetallsalzes durch Mischen und Erhitzen eines Fettsäureesters und einer Lösung, in der ein Alkalimetallsalz in einem Lösungsmittel gelöst ist,
wobei das in dem Schritt der Herstellung eines Saccharosefettsäureesters verwendete Fettsäurealkalimetallsalz in dem Schritt der Herstellung eines Fettsäurealkalimetallsalzes hergestellt worden ist.

5. Verfahren zur Herstellung eines Saccharosefettsäureesters nach einem der Ansprüche 1 bis 4, wobei in dem nachfolgenden Schritt das Erhitzen so durchgeführt wird, daß die Temperatur des Gemisches bei oder über einer Temperatur liegt, die 25°C unterhalb der Zersetzungstemperatur liegt und die unterhalb der Zersetzungstemperatur liegt.

6. Verfahren zur Herstellung eines Saccharosefettsäureesters nach einem der Ansprüche 1 bis 5, wobei in dem nachfolgenden Schritt das Erwärmen so durchgeführt wird, dass die Temperatur des Gemisches ansteigt.

7. Verfahren zur Herstellung eines Saccharosefettsäureesters nach einem der Ansprüche 1 bis 6, wobei die Erwärmungszeit des nachfolgenden Schritts 6 Stunden oder weniger beträgt.

## Revendications

1. Un procédé de production d'un ester d'acide gras de saccharose, comprenant :
une étape de préparation d'une solution aqueuse contenant du saccharose et un catalyseur basique ; et
une étape de production d'un ester d'acide gras de saccharose, en mélangeant la solution aqueuse obtenue à l'étape précitée, d'un sel de métal alcalin d'acide gras et d'un ester d'acide gras, et en chauffant le mélange avec agitation sous pression réduite,
l'étape de production d'un ester d'acide gras de saccharose ayant une étape antérieure consistant à éliminer l'eau du mélange, et une étape ultérieure consistant à effectuer une transestérification après l'étape antérieure, et
dans l'étape ultérieure, le chauffage est effectué par irradiation micro-ondes de telle sorte que la température du mélange soit inférieure à une température de décomposition du saccharose.

2. Le procédé de production d'un ester d'acide gras de saccharose selon la revendication 1, dans lequel il est produit un ester d'acide gras de saccharose avec une proportion pondérale de mono-ester de 60% en poids ou plus.

3. Le procédé de production d'un ester d'acide gras de saccharose selon la revendication 1 ou la revendication 2, dans lequel l'ester d'acide gras est un ester d'acide gras dont l'acide gras constitutif comprend au moins un acide gras choisi dans le groupe constitué par les acides gras saturés et insaturés ayant 8 à 24 atomes de carbone.

4. Le procédé de production d'un ester d'acide gras de saccharose selon l'une quelconque des revendications 1 à 3, comprenant en outre :
une étape de production d'un sel de métal alcalin d'acide gras en mélangeant et en chauffant un ester d'acide gras et une solution dans laquelle un sel de métal alcalin est dissous dans un solvant,
le sel de métal alcalin d'acide gras utilisé dans l'étape de production d'un ester d'acide gras de saccharose ayant été produit dans l'étape de production d'un sel de métal alcalin d'acide gras.

5. Le procédé de production d'un ester d'acide gras de saccharose selon l'une quelconque des revendications 1 à 4, dans lequel, dans l'étape ultérieure, le chauffage est effectué de telle sorte que la température du mélange soit égale ou supérieure à une température de 25°C inférieure à la température de décomposition et est inférieure à la température de décomposition.

6. Le procédé de production d'un ester d'acide gras de saccharose selon l'une quelconque des revendications 1 à 5, dans lequel, dans l'étape ultérieure, le chauffage est effectué de telle sorte que la température du mélange augmente.

7. Le procédé de production d'un ester d'acide gras de saccharose selon l'une quelconque des revendications 1 à 6, dans lequel le temps de chauffage de l'étape ultérieure est de 6 heures ou moins.
